# EUROPEAN PATENT SPECIFICATION

(11) **EP 3 256 201 B1**
(45) Date of publication and mention of the grant of the patent: **22.05.2024**
(21) Application number: 16749827.8
(22) Date of filing: 10.02.2016
(51) Int. Cl.: A61N 1/05, A61M 25/00, A61B 8/12, A61B 18/14, A61B 5/0215, A61B 5/145

(54) **LOW PROFILE MEDICAL DEVICE WITH INTEGRATED FLEXIBLE CIRCUIT AND METHODS OF MAKING THE SAME**
MEDIZINISCHE VORRICHTUNG MIT FLACHEM PROFIL MIT INTEGRIERTER FLEXIBLER SCHALTUNG UND VERFAHREN ZUR HERSTELLUNG DAVON
DISPOSITIF MÉDICAL À PROFIL MINCE DOTÉ D'UN CIRCUIT FLEXIBLE INTÉGRÉ ET SES PROCÉDÉS DE FABRICATION

(30) Priority: 10.02.2015 US 201514619021; 17.03.2015 US 201514660917
(43) Date of publication of application: 20.12.2017
(73) Proprietor: Cathprint AB, 114 19 Stockholm (SE); Källbäck, Bengt, 183 68 Täby (SE); Minar, Chris, New Prague, MN 56071 (US)
(72) Inventor: KÄLLBÄCK, Bengt, 183 68 Täby (SE); MINAR, Chris, New Prague, MN 56071 (US)
(74) Representative: FRKelly
(86) International application number: PCT/US2016/017403
(87) International publication number: WO 2016/130713

(56) References cited:
- WO-A1-2011/005165
- US-A- 5 857 974
- US-A- 5 938 694
- US-A1- 2007 032 722
- US-A1- 2007 219 551
- US-A1- 2007 219 551
- US-A1- 2009 143 651
- US-A1- 2009 240 249
- US-A1- 2010 094 279
- US-A1- 2010 305 420
- US-A1- 2013 066 194
- US-A1- 2013 225 965
- US-A1- 2014 249 520
- US-A1- 2014 276 789

## Description

### BACKGROUND OF THE INVENTION

### FIELD OF THE INVENTION

The invention relates to medical devices. More specifically the invention relates medical devices using flexible circuit technologies to create thin walled structures, having simplified manufacturability along with complex functionality and robust durability.

### BACKGROUND ART

Medical devices, such as catheters, guidewires, and sheaths are generally introduced into a patient through a needle inserted into a blood vessel such as an artery or vein and navigated to the area of interest or disease using fluoroscopy, MRI, ultrasound or similar tracking or visualization technology for guidance. Once at the area of interest these devices are used to diagnose and treat a variety of diseases such as cardiac electrical arrhythmias, coronary artery blockages, neurovascular artery aneurysms, as examples. The devices have dimensional requirements that require them to be small enough navigate in human vessels, organs, and cavities, in conjunction with other devices, while incorporating a growing number of sensors such as those needed for sensing pressure, temperature, location, movement, impedance, velocity, cell electrical activity, blood chemistry, images, acoustics and the like. In addition, some devices include conductors, pull wires, fiber optics, lumens, fluid lumens, stiffeners, braiding, structural elements and many other components typically found in such devices. In general, over several decades, these devices have developed, through clinical need, to be more sophisticated with more complex diagnostic and therapeutic capabilities and have also needed to be made in smaller sizes to fit into more complex, distal, and smaller anatomical regions, allowing for the treatment of significantly more tissue volume. United States Patent Publication No. 2009/0240249 teaches a probe for use in medical procedures that includes a flexible sheath and a flexible circuit. The flexible circuit is coupled to the sheath and also to an electrode and a thermocouple. United States Patent No. 5,857,974 teaches an ultrasound transducer assembly that includes a flexible circuit to which the ultrasound transducer array and integrated circuitry are attached. In an embodiment the transducer elements are arranged into a cylindrical array. United States Patent No. 5,938,694 teaches an electrode array catheter for mapping, pacing, and ablation that includes a flexible delivery sheath and an electrode assembly mounted within the delivery sheath, including electrodes and a flat flexible circuit having axially extending electrode traces connecting to electrode pads. United States Patent Publication No. 2010/0305420 teaches a flexible circuit that includes conductive material on the top and bottom planar surfaces of a dielectric substrate. The flexible circuit can be used as a sensor, and includes vias for electrical communication, and can be used in a medical instrument. WO 2011/005165 teaches a catheter with an external electrode connected via a conductor to an internal electrode. A narrow rectangular flexible printed circuit board is rolled into a tube and filled with an adhesive. US2009/240249 to Chan et al teaches a probe with a flexible sheath, a flexible circuit embedded in the sheath, and a thermocouple. US2007/219551 to Honour et al teaches a catheter or lead with a flexible printed circuit secured in a helical manner near or along the outside of the catheter. US2009/143651 to Kallback et al describes a device for invasive use with a support member comprising a flexible material. The support member has a layer of conductive lines. US2014/249520 to Ghaffari et al describes a flexible catheter or guidewire designed for flow monitoring. US2010/094279 to Kauphusman teaches a circuit for connecting an electrode to a catheter or sheath. The circuit is secured using reflow of the catheter.

However, the smaller the size of the device, the more difficult and expensive it is to manufacture, especially if it is made with an increasingly high density of electronic components, and other sophisticated elements. Complex assembly processes can be very complex and time consuming, especially when they are not automated, and as a result these medical devices are a relatively expensive burden on the health care system. There remains a need for a small, high performance medical device that is low in cost.

Devices using flexible circuit technologies to create thin walled medical device structures, having simplified manufacturability along with the desired complex functionality and robust durability, with pre-mounted smaller profile components, and less assembly time, would be well received in the medical marketplace.

Flexible circuits used in medical devices today are flat thermoset configurations. These circuits use materials, processes, sizes and basics designs common in the flexible circuit industry today and do not fit well with percutaneous introduced device designs and the processes to make them. What is needed are flexible circuit materials and configurations which are compatible with catheter, guidewire, and sheath designs and processes. Ideally the flexible circuit substrates could be made of materials like polyurethane, PEBAX, Polyester, and similar biocompatible thermoplastics, which can be reflowed into catheter shapes. It is also advantageous at times, when lubricity is important, to make substrate materials out of PTFE or other lubricious materials. It is also important for conductors in minimally invasive medical devices to be small in cross section, to keep the medical device size small. In all cases it is important when attaching conductive materials, such as copper, to these substrates, to get a strong and durable bond. It is also important to acquire the strong and durable bond without adding an adhesive layer which will increase the layer thickness. So a strong, durable, narrow and thin electrical conductor on standard disposable medical device substrates is highly desirable within the medical device industry.

### BRIEF SUMARY OF THE INVENTION

The present invention solves these needs by providing a medical device that includes an elongated lumen shaft that includes a shaft body having a shaft wall and as set out in the appended claims. The device also includes a cylindrical flexible circuit made of a dielectric layer in a cylindrical form, a conductive trace layer, a medical device element; and an open, unfilled lumen. In some embodiments the flexible circuit is integral to the shaft's mechanical structure. The cylindrical flexible circuit can have two or more substantially cylindrical flexible substrate layers and a via.

The via can provide fluid or electrical communication through one of the flexible substrate layers. The medical device element can be a sensor located on the interior of the cylindrical flexible circuit, and if so the device further includes an electrical connection between the sensor and the conductive trace layer through the via. The medical device element can be an electrode located on the exterior of the cylindrical flexible circuit, and if so the device further includes an electrical connection between the electrode and the conductive trace layer through the via. The medical device can have multiple medical device elements, one is located on the interior of the cylindrical flexible circuit, and a second medical device element located on the exterior of the cylindrical flexible circuit.

The medical device can include a flexible circuit edge joint that is sealed to form the cylindrical flexible circuit into a full cylinder. The joint can be longitudinally linear. The joint can also be longitudinally helical.

In one embodiment of the medical device the cylindrical flexible circuit is inside the lumen shaft, and the open, unfilled lumen is inside the cylindrical flexible circuit. Likewise, while the flexible circuit edge joint is sealed in one embodiment, in another the flexible circuit edge joint is not sealed, and the lumen shaft is adapted to hold the flexible circuit in a substantially cylindrical form.

In another embodiment the lumen shaft includes a sensor opening adapted to expose the medical device element to the exterior of the medical device.

In another embodiment the cylindrical flexible circuit is outside the lumen shaft, and the open, unfilled lumen is inside the lumen shaft.

In some embodiments the open, unfilled lumen has a pull wire or a fluid lumen. In some embodiments of the cylindrical flexible circuit it includes a cylindrical primary portion and a flat proximal portion. The medical device may also include a solder pad or a connector adapted to connect the cylindrical flexible circuit to external medical equipment.

In another embodiment the medical device includes an elongated lumen shaft that includes a shaft body having a shaft wall. The device also includes a cylindrical flexible circuit made of a dielectric layer in a cylindrical form, a conductive trace layer, and an open, unfilled lumen.

The disclosure also includes an unclaimed method of manufacturing a medical device that includes providing a flat flexible circuit that includes a dielectric layer, a conductive trace layer, and a medical device element; rolling the flat flexible circuit into a substantially cylindrical form; sealing the cylindrical flexible circuit into the substantially cylindrical form by substantially filling its lumen with an adhesive; providing an open lumen shaft on the exterior of the cylindrical flexible circuit; and removing the adhesive from the cylindrical flexible circuit to provide an open lumen. In one embodiment the method further includes the step of providing a sensor opening to expose the medical device element to the exterior of the medical device. In another embodiment the method further includes the step of soldering a proximal portion of the flexible circuit to a connector.

In another embodiment the flat flexible circuit is a spline or an arm of a larger catheter. In this embodiment multiple flat flexible circuits can be formed into catheter arms to form a basket catheter, an ray shaped mapping catheter, or a lasso shaped catheter. These arms can be soldered or attached to a larger, longer flexible circuit catheter body, or can be attached to a traditional catheter shaft.

In another embodiment the shaft has a body with a wall. The device also includes a cylindrical flexible circuit that includes a dielectric layer in a cylindrical form, a monomer layer covalently bonded to the dielectric layer, a conductive layer adhered to the monomer layer, a medical device element; and an open, unfilled lumen. The dielectric layer may include a thermoplastic, such as Pebax. In one embodiment the dielectric layer and the shaft body are substantially the same material.

In one embodiment the conductive layer comprises a seed layer and a trace layer. The seed layer can include a metal or metal ion selected from the group consisting of palladium, ruthenium, rhodium, osmium, iridium, platinum, silver, copper, and their ions, or a conductive polymer. When the seed layer is a conductive polymer, the seed layer and the monomer layer can include the same monomer, or different monomers copolymerized.

In one embodiment the trace layer includes a metal or metal ion selected from the group consisting of copper, silver, gold, nickel, titanium and chromium. The seed layer and the trace layer can include a combination of the same or different metal or ions thereof.

In another embodiment the medical device's monomer layer is covalently bonded to the dielectric layer in a desired conductor pattern. The monomer layer may include a carboxylic group.

In one embodiment the medical device may include a conductive trace that is between 5 um and 20 um wide. The flexible circuit can be between 1 um and 30 um thick, and can exclude any adhesive layer between the dielectric layer and the conductive trace layer.

In another embodiment the medical device includes an elongated lumen shaft that includes a cylindrical flexible circuit that has a dielectric layer in a cylindrical form, a monomer layer covalently bonded to the dielectric layer, a conductive trace layer adhered to the monomer layer, a medical device element, and an open, unfilled lumen.

The disclosure further includes an unclaimed method of manufacturing a medical device that includes the steps of preparing a flat flexible circuit by providing a flexible substrate comprising a dielectric layer, covalently bonding a monomer layer to the dielectric layer, adhering a seed layer to the monomer layer, coating the seed layer with a conductive trace layer, adding a medical device element, rolling the flat flexible circuit into a substantially cylindrical form, sealing the cylindrical flexible circuit into the substantially cylindrical form by substantially filling its lumen with an adhesive; and removing the adhesive from the cylindrical flexible circuit to provide an open lumen.

The method may include the step of covalently bonding a monomer layer to the dielectric layer comprises the substeps of adding a photo initiator, and activating the photo initiator in a desired conductor pattern or a negative of a desired conductor pattern. The method may also include the step of activating the photo initiator with laser energy.

### BRIEF DESCRIPTION OF THE DRAWINGS

FIG. 1 is a sectional view of a flexible circuit used for the invention;
FIG. 2 is a sectional view of a flexible circuit cross section which may be used for the invention;
FIG. 3 is a cross section of a flexible circuit configured as part of an elongated open lumen body with the flexible circuit disposed on the inside;
FIG. 4 is a cross section of a flexible circuit configured as part of an elongated open lumen body with the flexible circuit disposed about the outside diameter;
FIG. 5 is a cross section of a flexible circuit configured into an elongated open lumen body;
FIG. 6 is an isometric view of the manufacturing of the device;
FIG. 7 is a schematic drawing showing one embodiment of a manufacturing tool;
FIG. 8A is a perspective view of a flexible circuit during construction;
FIG. 8B is a perspective view of a flexible circuit during construction;
FIG. 9A is a perspective view of a flexible circuit during construction;
FIG. 9B is an perspective view of a flexible circuit during construction;
FIG. 10A is an perspective view of a flexible circuit during construction;
FIG. 10B is an perspective view of a flexible circuit during construction;
FIG. IOC is an perspective view of a flexible circuit during construction;
FIG. 10D is an perspective view of a flexible circuit during construction;
FIG. 11 is a view showing the flexible circuit batch;
FIG. 12 shows a perspective view of manufacturing;
FIG. 13 shows an isometric view of manufacturing;
FIG. 14 shows a cross section of one embodiment of the manufacturing process for constructing the invention.

### DETAILED DESCRIPTION OF THE INVENTION

In general, the invention comprises a medical device used for diagnostic and/or therapeutic surgical procedures. For example, the device could be a guidewire, a catheter, a sheath, or other medical device to be inserted into a patient.

In one embodiment, the device includes an elongated open lumen body. An elongated open lumen body is an elongated tubular structure which is not filled with structural material. It is open so that other elements, e.g., pull wires, fibers, conductors, additional lumens, stiffeners, or fluid, may be run from one end of the medical device to the other or through a portion of the device. An "open" lumen can be capped or sealed at the ends or in a portion thereof. In another embodiment the device includes an elongated closed lumen body that is filled with material, including for example structural material or adhesive. In another, separate embodiment the device includes an elongated flexible circuit that is placed within a separate elongated tubular structure.

As part of this invention, the device has a structure that includes a flexible circuit comprising one or more dielectric layers (such as polyimide, silicone, parylene, LCP, , reinforced composites, for example); and one or more electrically conductive layers (such as copper, silver, carbon, conductive inks, for example); and possibly one or more mounted electronic components (such as electrodes, thermistors, capacitive micromachined ultrasonic transducers, pressure sensors, for example), which may be mounted in, on or within the elongated open lumen body over part or the whole of its length. Flexible circuits are known in the industry under a variety of names, including flexible printed wire boards (PWB), flexible electronics, flexible printed wiring, flexible printed circuit board (PCB), flexible printed wire assembly (PWA), flexible printed circuit assembly (PCA), or flexible printed circuit board assembly (PCB A). While in some settings there are slight differences between these terms, for purposes of this invention the term flexible circuit board will be used to encompass flexible or conforming boards with a wiring and with or without mounted sensors or other components.

As an alternative to or in addition to the electrically conductive layer, the flexible circuit may comprise a flexible integrated photonics layer, a flexible silicon photonics layer for use, for example, as an interferometer or resonator. Alternatively the photonic and electronic layers may be combined into one layer.

Instead of lying flat, one or more of the flexible circuit layers is rolled or partially rolled so that at least a portion of its edges abut each other or overlap (with each other or the ends of a another flexible circuit) to give the elongated open lumen body a seam or a joint, for example a lap joint. These joints may be held together with an adhesive, a reflowed substrate made of a thermoplastic, encapsulation within other layers, or by a mechanical means. In some embodiments the ends have a substantial gap between them that is filled or held in place by a thermoplastic, for example, In a closed lumen embodiment, the joint may be held together by an adhesive that fills the lumen. In other embodiments, the seam is sealed or joined by a laser, e.g., creating a laser welded seam.

This lumen body can contain electrical conductors and sensors but also may have strengthening members within its layer (such as carbon fiber, stainless wire, for example), and also may contain pull wires, optical fibers, fluid lumens, and electrical wires. These components may lie at the center of the rolled layer, e.g. within a lumen and over at least part of the length from proximal and distal ends. In this configuration of the invention the flexible circuit acts as a structural component carrying the device's mechanical load such that the balance of the medical device construction can be reduced in cross-section and thus reducing total wall thickness and total device diameter, allowing for less traumatic procedures and exponentially improved access to distal tissues of interest. In other configurations, the reduced cross section allows for a larger lumen that allows additional components in the lumen.

In an exemplary embodiment of the invention, a flexible circuit, over all or part of its length is rolled into a tubular, or partial tubular, shape such that the seam or edges run longitudinally down the length of the shaft, covering all or part of the circumference of an open lumen tubular shaft material such as a stainless hypo tube, or a polymer catheter shaft, for example. The seam or edges are affixed together as described above, such as by means of an adhesive or a thermoplastic reflow process, for example. As background information useful for understanding the invention, a thermoset dielectric may be induced to hold a tubular or partial tubular shape through use of a stress relieving process. The latter may serve to hold the edges of the flexible circuit together over its length, but both the flexible circuit or the open lumen tubular shaft material may comprise the load bearing structure of the invention. According to the invention, both flexible circuit dielectric material and the tubular shaft material are made of a thermoplastic and reflowed together, for additional strength. In an alternative mode of this and other embodiments, the seam or edges may run in a spiral configuration about the shaft. Likewise, the seam or edges may be circumstantially offset at different portions of the shaft. That is, in a first proximal portion of the shaft the seam may be at 12:00 as looking at the cross-section of the shaft, in a middle portion the seam may be at 4:00, while in a distal portion of the shaft the seam may be at 8:00. This pattern may happen once or be repeated, as needed to either distribute the seam to reduce weakness or biasing in any one direction, or to increase weakness or bias in a direction at a particular location.

In another embodiment of the invention, a flexible circuit, over all or part of its length, is rolled into a tubular, or partial tubular shape such that the seam or edges run longitudinally down the length of the shaft, is placed inside of an additional tubular shaft material made of a metal tube or braided polymer shaft, as an example. The latter may serve to hold the edges of the flexible circuit together over its length. In one embodiment the flexible circuit does not cover the whole circumference of the tube. In one mode of this embodiment both the flexible circuit and the additional shaft material comprise the load bearing structure of the invention. The flexible circuit may be fitted into an existing tubular shaft and held in place by mechanical force, adhesive, extrusion, or shrink tubing, for example, or, in accordance with the invention, by thermoplastic reflow. For example, the flexible circuit may be affixed to a mandrel, placed inside a cylinder, e.g., a shrink tube, released from the mandrel, have the mandrel removed, and then be shrunk to its final diameter by the shrink tube. A similar design may be achieved by first creating the tubular flexible circuit structure then dip coating or spray coating the flexible circuit structure. Alternatively or in combination with the above a thermoset dielectric may be shaped with a stress relieving process.

The previously described embodiments may also be combined as needed over the length of the device to create a hybrid or composite device.

The device has a greatly simplified construction compared to the prior art devices. Briefly, a flexible circuit is manufactured having on it the necessary traces, electrodes, connections, sensors, and vias for the device. This flexible circuit may then be wrapped around a core and the seam sealed (or not, as discussed above). It may be affixed, e.g., glued, to the core or to a portion of the core, giving the assembly a cylindrical shape. The core may then be removed to open up the lumen. Likewise, the flexible circuit can be wrapped around a removable core, placed inside a second tube and then have the core removed to open up the lumen. The removable core may be coated with a lubricious coating or stretchable such that upon stretching the core's OD shrinks allowing it to be removed from the assembly exposing the open lumen. In an alterative the flexible circuit can be wrapped around a hollow open lumen and glued to itself or the tube.

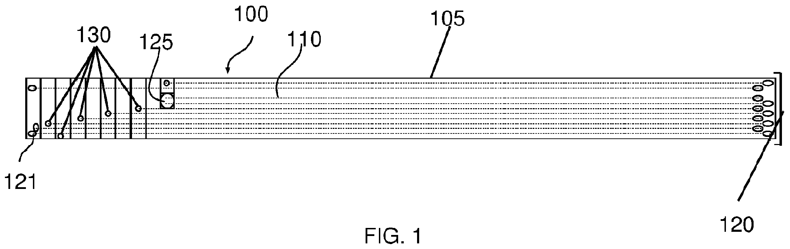

FIG. 1 shows an exemplary (flat) flexible circuit 100 for use as a basic building block of the medical devices of the present invention. A flexible circuit 100 utilizes a flexible substrate 105, typically made with a thin flexible plastic or metal foil as the substrate. The flexible circuit 100 is advantageously long, thin and narrow. Often, the flexible substrate 105 utilizes an insulating material or a dielectric. The substrate is. made for example from thermoplastic polymers. The substrate can be made from polymers such as liquid-crystal polymers (LCP), polyether ether ketone (PEEK), polyester, Polyethylene terephthalate (PET), polyimide (PI) (e.g., DuPont Pyralux^{®}), polyethylene napthalate (PEN), polyetherimide (PEI), polyefine, Kapton, various fluropolymers (FEP), PTFE, silicone, parylene, reinforced composites and copolymer Polyimide films or a transparent conductive polyester film or other dielectrics. One non-limiting example of the latter is the product Topas^{®} COC (TOPAS Advanced Polymers GmbH, Oberhausen, Germany or Ticona GmbH, Kelsterbach, Germany). In some cases the flexible substrate 105 advantageously includes a first polymer coated by a second polymer that provides superior adhesion to the next layer, for example parylene, by providing for example covalent bonding sites as discussed in detail below.

Advantageously the material for the flexible circuit 100 is biological -inert or bio-compatible. The flexible circuit 100 may also be covered with a layer of biocompatible hydrogel, silicone, PTFE, for example on the outside to reduce the friction and for improved biocompatibility, e.g. to avoid blood coagulation.

The measures of the flexible circuit 100 may in one advantageous embodiment be 100 cm long, 1.5 mm wide and 50 µm thick. The length, as well as the thickness and the width, vary depending on the application. The width may be in the interval of 0.5-10 mm, more advantageously 1-5 mm, and the thickness may be in the interval of 2-200 µm, more advantageously 3-50 µm, in one particular embodiment 50 µm is used. Generally a greater thickness results in a more rigid device and a smaller thickness results in a less rigid one. A thinner material, e.g. a laminate below 25 µm with conductive traces thinner than 25 µm, is typically more flexible, but in embodiments where increased rigidity is required the thickness is increased along all or a portion of the substrate 105. In embodiments with an adhesive the adhesive may add 10 um to 50um to the thickness of the flexible circuit 100.

In some cases, closed lumen medical devices can become too stiff when the diameter becomes large, as they do not have an open lumen. In this case, the stiffness of a cylindrically shaped closed lumen device is proportional to the fourth power of the diameter. Thus, the larger the device's diameter, the substantially larger the stiffness will be. For a diameter below 1mm the catheter is soft and flexible. However, for such a closed lumen device it is very difficult to make it very flexible. In one embodiment the present invention solves this difficulty by creating an open lumen and using the flexible circuit 100 as all or part of the structural element, reducing or eliminating the need for bulky polymer walls. Notably, it may be desired to create a region of the medical device that is more susceptible to bending or other faults, and accordingly such a region may have a thinner or otherwise modified flexible circuit substrate. Similarly, this region may use the flexible circuit of the present invention while other portions of the device rely on traditional catheter technology.

Conductive traces 110 are formed onto substrate 105. For example, a metal foil layer may be applied to or adhered to the substrate 105. Conductive traces 110 may be etched from this foil layer. Traditionally a copper foil is used, but a wide variety of foils of varying materials (metals, alloys, conductive polymers) thicknesses, conductivities, and cost are available. A thin polymer coating (not shown) may be applied over the conductive traces 110. The conductive traces 110 may be formed of a metal such as silver or copper, conductive inks and adhesives, conductive fiber such as carbon. They may be constructed by photolithography, conductive ink aerosol ink jet printing, sputter coating, etching, rolling, electroplating, vapor deposition or other methods known in the art.

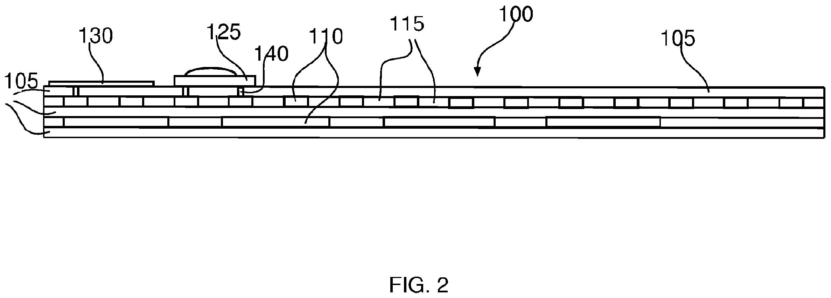

Conductive traces 110 may be arranged on both sides of the substrate 105. At certain points there are holes, called via holes 140 (see FIG. 2) in the substrate 105. Conductive traces 110 on opposite sides of the substrate 105 are electrically connected through the via holes 140. In the via holes 140 there is electrically conductive material on the walls. The conductive traces 110 may comprise a suitable metal, e.g. copper or an electrically conductive polymer or another electrically conductive material. In one embodiment a conductive trace on a first side of substrate 105 connects to a component on the opposite or second side of substrate 105, such as solder pad 120, 121, sensor 125, or electrode 130, preferably through via hole 140, but alternatively by other means such as a trace through substrate 105, or an electrically conductive portion of substrate 105.

The flexible circuits utilized in the present invention may be single sided, double sided, double access, sculptured, or multilayer flexible circuits. Single sided circuits have the advantage of being easy to manufacture. They have a single conductive trace layer formed on one side of the substrate.

Double access flexible circuits likewise typically have a single conductive trace layer, but are further processed so that portions of the conductive trace layer are accessible from both sides for ease of connection to a sensor, electrode, or the like. Double sided flexible circuits typically have two conductive trace layers, one on each side of one or more substrate layers. They are often advantageously constructed with through holes, or vias, to provide connection features for the conductive traces on one or both sides of the substrate. The present invention also contemplates the use of multilayer flexible circuits, which may have any number of substrate layers and conductive trace layers, the latter of which may be interconnected by vias.

Likewise, the present invention takes advantage of a stretchable flexible circuit, allowing the device to take on various curvilinear shapes, bends, and motions during use. Such a stretchable flexible circuit can be especially useful for a catheter that must conform to physical anatomy, or for use in a catheter portion that is inflated and deflated during use. When a stretchable dielectric is used the conductor material is also ideally stretchable, such as an elastic conductive polymer, or a metal shaped in such a way to be stretchable, e.g., in a serpentine, zig zag, rippled, or otherwise elongatable pattern. Examples of stretchable elastomers used in substrate material include polymeric organosilicon compounds (commonly referred to as "silicones"), including Polydimethylsiloxane (PDMS), certain polyimides; photopatternable silicone; SU8 polymer; PDS polydustrene; parylene and its derivatives and copolymers (parylene-N); ultrahigh molecular weight polyethylene; poly ether ether ketones (PEEK); polyurethanes (PTG Elasthane^{®}, Dow Pellethane^{®}); polylactic acid; polyglycolic acid; polymer composites (PTG Purisil Al^{®}, PTG Bionate^{®}, PTG Carbosil^{®}); silicones/siloxanes (RTV 615^{®}, Sylgard 184^{®}); polytetrafluoroethylene (PTFE, Teflon^{®}); polyamic acid; polymethyl acrylate; stainless steel; titanium and its alloys; platinum and its alloys; and gold. In embodiments, the substrate is made of a stretchable or flexible biocompatible material having properties which may allow for certain devices to be left in vivo.

The proximal end of the conductive traces 110 may be terminated in a connective means such as a solder pad 120, connectors, or similar structure, for connecting the trace to other medical equipment, such as a power source, diagnostic equipment, or monitoring equipment. The distal end of the conductive traces 110 are terminated in medical device elements, such as sensors 125, electrodes 130 or distal solder pads 121, for example. Parameters that may be measured include pressure, temperature, flow, pH, partial pressure of oxygen, mapping with ultra sound etc. It is also possible to combine different electronic components and/or microelectromechanical systems to achieve multi functionality or to integrate several electronic components or microelectromechanical systems of one kind to get extended functionality. One such example could be several pressure sensors in order to improve diagnosis of stenosis in the coronary arteries. Distal solder pads 121 may connect to a set of proximal solder pads 120 on a second flexible circuit 105, or may connect to a set of solder pads 120 on a traditionally formed catheter segment or component.

While this application discusses solder pads as a common connection technique, it should be understood that various connection methods are contemplated. For example, neurological leads and pacemaker/ICD leads have several common connection techniques, such as male and female IS-1 connectors, LV-1 connectors, and the like. It is contemplated that the present invention can be utilized to form such connectors, or have them attached to the flexible circuit. Due to the extreme need for a smaller diameter and extreme flexibility, the present invention is particularly suited to neurological leads and cardiac leads. Such devices can be manufactured via any of the means disclosed above or below.

When the medical device elements have been mounted, the flexible circuit 100 is at least partly rolled up into a tube and may be simultaneously filled with adhesive or glue that holds the flexible circuit 100 in a tube shape. Formation of the flexible circuit 100 at least partly into a tube is advantageously done by feeding the flexible circuit 100 through a hole with a funnel-like opening where the circumference of the hole matches the width of the flexible circuit 100. When a single sided flexible circuit 100 is used it is advantageous that the width of the flexible circuit 100 is the same as the circumference of the hole. When a double sided flexible circuit 100 is used it is advantageous that the width of the flexible circuit 100 is slightly smaller than the circumference of the hole. This is necessary because elements on the second side of the flexible circuit 100, such as the medical device elements or the conductive traces 110 need some space in the hole. After feeding the flexible circuit 100 through the hole, the first and second side of the flexible circuit 100 have respectively become inside and outside of the substantially cylindrical flexible circuit 100.

After processing, the flexible circuit 100 is, alone or with other flexible circuit(s) 100, in a substantially cylindrical shape. That is, the boundaries of the flexible circuit 100 - while not necessarily contiguous or closed - define a generally hollow tubular shape that is substantially cylindrical. A cylinder is the surface generated by a straight line intersecting and moving along a closed plane curve, the directrix, while remaining parallel to a fixed straight line that is not on or parallel to the plane of the directrix. An exemplar cylinder is bounded on the top and bottom by flat circular ends and by a single curved side. However, the cylindrical shapes of the present invention, because they are real world devices and not pure mathematical constructs, will not have perfectly circular tops and bottoms, but in fact may be irregular or in another form, e.g., an oval. Likewise, the single curved side may not be straight, even during manufacturing. During use of the medical device it is required to bend and twist to reach its target. Viewed in two dimensions one side may not match the other. While the cylindrical shape may be a right circular cylinder in some embodiments, in others it will be an oblique cylinder, In open lumen devices, the flexible circuit 100 may have a closed top and bottom, but in a preferred open lumen embodiment the cylinder is an annular cylinder or a tube, and the top and bottom are in fact open allowing the passage of fluids, wires, and the like. Within this understanding, the flexible circuit 100 is formed from a flat flexible circuit into a substantially cylindrical shape for use in the medical device.

FIG. 2 shows a lateral cross section of one embodiment of a flexible circuit 100 suitable for use in the present invention. As shown therein, flexible circuit 100 comprises multiple flexible substrate layers 105, which can be comprised of one or more similar or different polymers or other dielectrics which are flexible enough to be used in a medical device. The flexible circuit 100 also contains multiple conductive trace layers 110. These layers may or may not be held together with an adhesive 115. Likewise, an adhesive 115 or polymer may fill any gaps between traces. The proximal end of the conductive traces 110 are ideally terminated into a connector by means of a solder pad 120, connector, or similar (not shown). The distal end of the conductive traces 110 are terminated into distal medical device elements such as sensors 125, electrodes 130, solder pads 121, diagnostic devices (e.g., thermistors, pressure sensors, glucose monitors, etc.), or therapy devices (e.g., an ultrasound array, and ablation element, laser, cryogenic fluid delivery, etc.) The distal end of the conductive traces 110 may terminate to the distal element (125, 130, 121) by means of vias 140, for example. The vias 140 may act as pathways through a dielectric layer 105 for a continuance of a conductive trace 110, a connection to an electrode 130, a connection to a sensor 125, connection to a solder pad 121, for fluid transmission between layers, inflation of a balloon, or a combination of these. For example, a via 140 may provide a pathway through the dielectric layer 105 for an electrical connection between sensor 125 and one of the conductive trace 110, and also serve as a fluid lumen for one or more purposes, such as irrigating tissue, cooling a sensor 125 or an ultrasound array (not shown), or balloon inflation and deflation. In one embodiment, the conductive trace 110 may be formed on the inside of the tube and the electrodes 130 or sensor 125 may be formed on the outside of a single double sided flex board based on a thermoplastic substrate without having an inner supporting tube, and be connected by via 140. The conductor lines could be copper and the electrode platinum, for example, or another combination. The medical device elements may be clamped or secured to the flexible circuit using a collar (not shown). The medical device elements may also be attached to the flexible circuit substrate using bond pads (not shown).

The flexible circuit 100 may be comprised of one or more dielectric layers 105 and one or more conductive trace layers 110. Each layer may be fractions of a micron thick as long as they satisfy the electrical requirements of the device. Distal elements (e.g., electrodes, solder pads and sensors, etc.) may be disposed on any layer of the flexible circuit 100 and on either side of the dielectric layers 105. While three dielectric layers 105 are shown, and two conductive trace layers 110 are shown, it is understood that other combinations are within the scope of the invention.

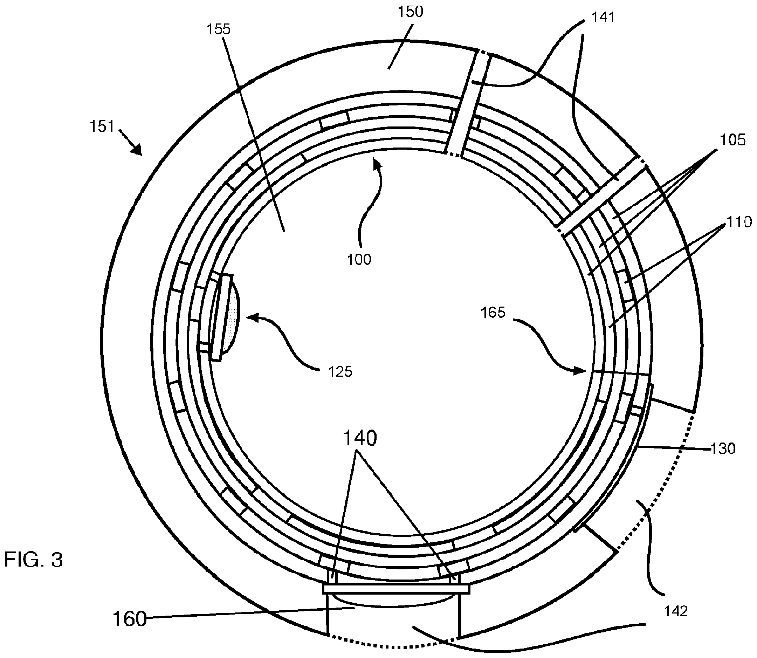

FIG. 3 shows a medical device 151 used for diagnostic and/or therapeutic procedures, in the form of an elongated body 150, such as a guidewire or catheter body. Elongated body 150 has an open lumen 155. The elongated body 150 may be constructed of a polymer extrusion with or without reinforcement, a shrink tube such as polyester or PTFE, a polymer structure formed through dip coating, a polymer structure formed from reflowing of a polymer, or a metal tubular structure such as a hypo-tube. In some embodiments, particularly for a guidewire, neurological lead, or cardiac lead, the lumen is absent, e.g., a guidewire wherein the flexible circuit is wrapped so tightly that there is practically no lumen space remaining between opposite cylinder walls. In such an embodiment the flexible circuit may be wrapped in an overlapping, e.g., spiral pattern. Of note, there would still be an insignificant gap where the spiral pattern starts, unless the flexible circuit substrate was formed of a material, e.g., a reflowable material that could be manipulated to fill that gap. Such a reflowable material could fill in any lumen or be used to smooth out the device, fill in around attached ring electrodes and sensor. Such electrodes may be attached via traditional means, via vapor deposition, sputtering, or evaporation techniques, and may be constructed of copper, silver, gold, platinum, or titanium materials.

Flexible circuit 100 is mounted inside of lumen body 150, in open lumen 155. In one embodiment lumen body 150 is formed around a cylindrical flexible circuit 100. For example, lumen body 150 may be reflowed over the already rolled and cylindrical flexible circuit 100 (as discussed above). Likewise, a shrink tube may be placed over the cylindrical flexible circuit 100 and shrunk to fit in place.

In the alternative, flexible circuit 100 may be slid into lumen body 150 and adhered into place by one or more mechanisms, such as an adhesive, a filler polymer, shrinking lumen body 150, crimping, and the like. The flexible circuit 100 may be "over rolled" as discussed above, e.g., it may be rolled to a smaller diameter than desired in the end product. It may then be slid into the lumen body 150, and the adhesive holding the edges of flexible substrate 105 together may be removed, allowing it to expand into the desired cylindrical shape.

In one embodiment, the flexible circuit 100 is placed onto a mandrel or rod and glued into place. It can also be heat formed or mechanically held on the mandrel. It is then placed inside the lumen body 150. At this point the mandrel is removed by melting the glue. In the alternative the mandrel may be elongated to decrease its diameter, and then removed. For example, the mandrel may be coated with a lubricious surface like PTFE or silicon. Likewise, the mandrel can be made from a material that can handle high temperatures, can be stretched, and necked down in OD, such as an annealed stainless steel, copper, etc.

In some embodiments the flexible circuit 100 is formed into its cylindrical shape without the mandrel. For example, it can be heat shaped into the tubular or semi-tubular shape and then possibly glued or mechanically held in position. As needed, the glue and mechanical constraints can be removed once flexible circuit 100 is inside the lumen body 150. Likewise, the flexible circuit 100 can be drawn or pulled into the lumen body 150 and held in place by a mechanical bias outward, with adhesives, by reflowing the outer shaft material to adhere or hold the flexible circuit 100, or any combination thereof.

Flexible circuit 100 may include one or more flexible substrates 105, for example, and one or more electrically conductive layers or elements 110, such as copper, silver, gold, platinum, titanium, carbon, conductive inks, for example, and possibly one or more mounted electronic elements, such as electrodes 130, thermistors 125, capacitive micromachined ultrasonic transducers 160, pressure sensors, for example, which may be mounted in, on or within the elongated open lumen body over part or the whole of its length. The edges of the flexible substrate layers 105 may or may not meet or overlap to form a flexible circuit edge joint 165, e.g., with a lap joint or butt joint. The flexible circuit edges may be held together with an adhesive, a reflowed substrate made of a thermoplastic, laser welding, or by mechanical means. The flexible circuit 100 is held in place within the open lumen 155 using the following: melting of a thermoplastic polymer dielectric to the inner wall of the medical device and melting the open lumen 155 to the flexible circuit.

The flexible circuit 100 may be formed of multiple or variable widths, as to fill the inner circumference of the open lumen 155 as the inner diameter of the open lumen 155 may vary over its length and require both edges of the medical device meet at a flexible circuit edge joint 165, and also to accommodate non-fully circumferential solutions where the requirement is for flexible circuit edges have a gap between then. The lumen 155 is depicted as being round in cross section, but may have other shapes as well, such as an oval or an irregular shape where needed. The flexible circuit 100 may be placed into the open lumen 155 such that the flexible circuit edge runs in a helix pattern over the length of the medical device lumen or in a single nonrotating fashion over its length. The mounted electronic components may be mounted on either side of the flexible circuit as shown in FIG. 3 by the sensor 125 on the inner portion of the flexible circuit (in the open lumen 155) and the electrode 130 on the outer portion. Irrigation ports 141 may facilitate fluid or pressure communication from the inner diameter to the outer diameter of the elongated lumen body 150. Sensor openings 142 may also be sued to facilitate communication between sensors 125, electrodes 130, and a target, such as a portion of a patient.

In this embodiment the elongated open lumen body 150 with inner mounted flexible circuit 100 may be used as the main medical device structure which not only contains electrical conductors and sensors but also may have strengthening members within its layer, such as carbon fiber or stainless wire. It also may contain pull wires, optical fibers, fluid lumens, and electrical wires and over at least part of the length from proximal and distal ends. In this configuration of the invention the flexible circuit 100 acts as part of the structural component carrying all of the device's mechanical loads such that the balance of the medical device construction can be reduced in cross-section, thus reducing total wall thickness and total device diameter, allowing for less traumatic procedures and exponentially improved access to distal tissues of interest.

In addition, the lumen body 150 may be the entirety of or a part of a single lumen catheter shaft. It may be one shaft and lumen of a multiple lumen catheter shaft, a sheath lumen, a guidewire lumen, a lumen forming part of an diagnostic or therapeutic assembly at the distal end of a device such as a catheter, or other medical device to be inserted into a patient, for example. The lumen body 150 may be a portion of an ultrasound catheter, a guidewire, an endoscope, a therapy catheter, a diagnostic catheter, or an OCT/OCR catheter or guidewire.

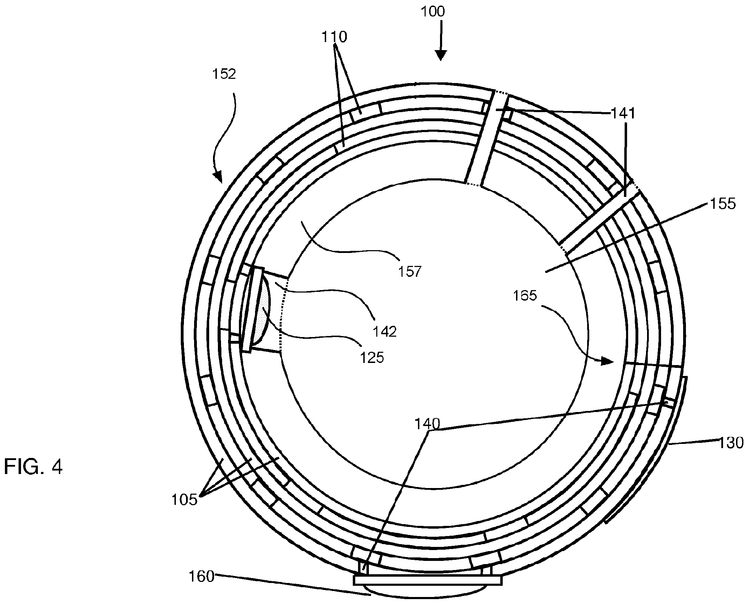

FIG. 4 depicts another embodiment of the invention, 152. In the embodiment shown in FIG. 4 the cylindrical flexible circuit 100 is outside open lumen shaft 157. The medical device 152 of this embodiment is used for diagnostic and/or therapeutic surgical procedures. It has the cylindrical flexible circuit 100 placed onto the open lumen shaft 157 and includes an open lumen 155. The cylindrical flexible circuit 100 is adhered to an open lumen shaft 157, for example using an adhesive, melting a thermoplastic polymer dielectric to the open lumen shaft 157, laser welding, or melting the open lumen shaft 157 to the flexible circuit.

The medical device 152 may be a single lumen catheter shaft, a lumen of a multiple lumen catheter shaft, a sheath lumen, a guidewire lumen. The medical device 152 may serve as a part of a diagnostic or therapeutic assembly at the distal end of a device such as a catheter, sheath or guidewire, with the remainder of the device formed by conventional means. The open lumen 155 may contain electrical conductors and sensors, strengthening members, such as carbon fiber, stainless wire, for example, and also may contain pull wires, optical fibers, fluid lumens, and electrical wires, within the open lumen and over at least part of the length from proximal and distal ends. One or more of these elements may be embedded in the shaft (150 or 157) or flexible circuit 100 as well.

The structure of the open lumen shaft 157 material may be constructed of one or more of the following; a polymer extrusion with or without reinforcement, a shrink tube such as polyester or PTFE, a polymer structure formed through dip coating, a polymer structure formed from reflowing of a polymer, a metal tubular structure such as a hypo-tube, for example. The flexible circuit 100 may be formed of multiple or variable widths, and placed on the outer circumference of the open lumen shaft 157, as the outer diameter may vary over its length and may require both flexible circuit edges 165 to meet, and to also accommodate non-fully circumferential solutions where the flexible circuit edges have a gap between then. The cylindrical flexible circuit 100 may be placed onto the open lumen shaft 157 such that the flexible circuit edge runs in a helix pattern over the length of the medical device lumen or in a single nonrotating fashion over its length. The cylindrical flexible circuit may have sensors 125, electrodes 130, or transducers 160 mounted on either side of the flexible circuit. Sensor openings 142 may facilitate communication to sensors and electrodes or contact with a target.

Manufacturing such a structure may be accomplished by either; first mounting the open lumen shaft 157 onto a removable carrier then mounting the flexible circuit 100 to the open lumen shaft 157 then removing the carrier, or by placing the flexible circuit 100 onto the open lumen shaft 157 without a carrier. The flexible circuit 100 can be glued into place, or it can be held in place by reflowing the lumen shaft 157 to hold the flexible circuit 100. Vias 140, irrigation ports 141, or sensor openings 142 may be mechanically formed, or created by use of a laser, before or after the flexible circuit 100 is mounted.

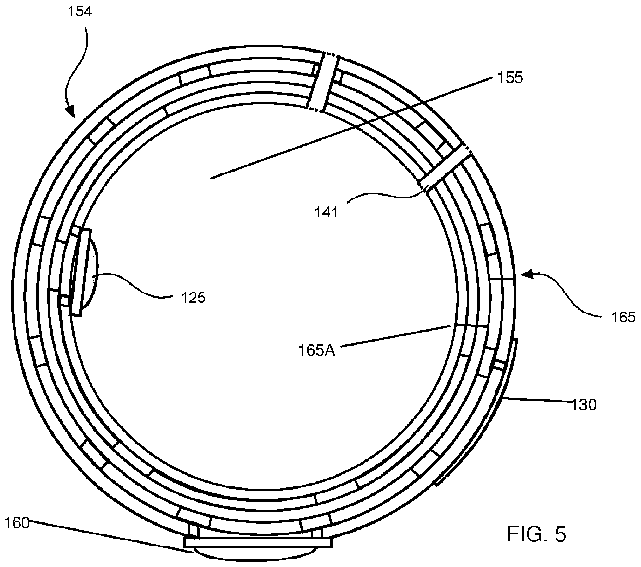

FIG. 5 shows another embodiment of the present invention in which the flexible circuit 100 itself forms the length of the elongated open lumen body 154, without the support of additional structural elements (beyond that of a non-structural seal or biocompatible coating). In this embodiment the flexible circuit edges 165 of the flexible circuit 100 are joined by one or more of the following; gluing a butt or lap joint together, laser welding, reflowing a thermoplastic dielectric, joining edges made of an interlocking pattern.

The elongated open lumen body 154 may form a single lumen catheter shaft, a multiple lumen catheter shaft, a sheath lumen, a guidewire lumen, or a lumen forming part of a diagnostic or therapeutic assembly at the distal end of a device such as a catheter, sheath or guidewire, for example. The flexible circuit 100 may be formed of multiple or variable widths, as the outer diameter of the elongated open lumen body 154 may be required to vary over its length. The flexible circuit 100 may be oriented over the length of the elongated open lumen body 154 such that the flexible circuit edge runs in a helix pattern over the length of the elongated open lumen body or in a single nonrotating fashion over its length, or in combination. Elements such as flexible circuit sensors 125, electrodes 130, or transducers 160 may be mounted on either side of the flexible circuit. Vias 140 may facilitate communication from the inner diameter to outer diameter of the elongated open lumen body 154, or between any given layers of flexible circuit 100.

The open lumen 155 may contain electrical conductors and sensors, strengthening members, such as carbon fiber, stainless wire, for example, and also may contain pull wires, optical fibers, fluid lumens, and electrical wires, within the open lumen and over at least part of the length from proximal and distal ends.

The open lumen 155 may be formed by wrapping a flexible circuit 100 around a mandrel and or gluing the joint together, e.g., a lap joint glued together. Likewise, it may be formed by reflow or melting the edges together with a thermoplastic polymer substrate. The mandrel or glue may be removed as described above, as needed, to form the open lumen. Likewise, in each of the embodiments disclosed, there could be a combination of layers and embodiments, for example a flexible circuit layer inside an shaft layer, and a second flexible circuit layer outside of the shaft layer.

It is also anticipated that hybrid or composite devices may be made by combining the structure described in these individual embodiments, such that the structure of the open lumen body varies over its length and diameter to fit specific needs of the designer, manufacturer, and user.

The catheter components described above can be used to form numerous types of devices, e.g., catheter, guidewire, or leads, in any number of different designs. In particular, a component formed by one embodiment above, may be combined with a component formed by any of the other embodiments to result in a device that meets a particular need. So, for example, a flexible circuit with a traditional pebax catheter layer around it, as described above, may form a long catheter shaft. A second catheter component may then be attached at the proximal end of this catheter shaft formed of a thin cylindrical flexible circuit layer without a pebax layer (on the inside or outside) to form a more flexible bend region. Alternatively, this second component may have a thinner layer of standard catheter wall material (pebax as an example) on the inside or outside. This catheter may then have a third component formed in a similar or different manner than the first or second components.

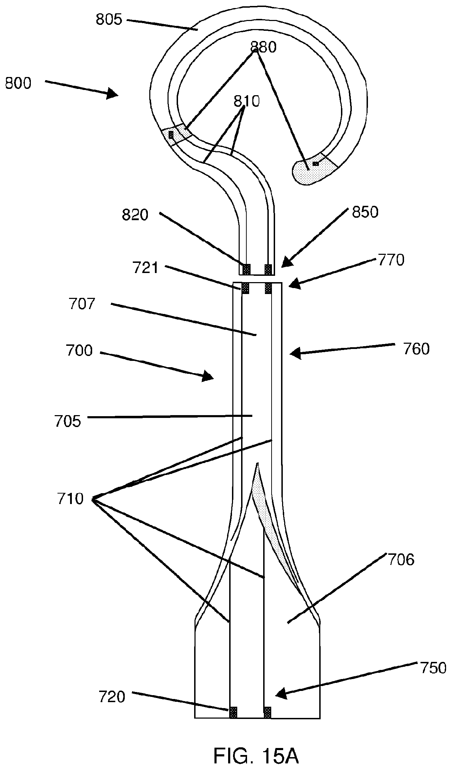

So, in one embodiment, shown in Figs. 15A-C, a first catheter component comprises a flexible circuit 700 with a flexible substrate 705, typically made with a thin flexible plastic or metal foil as the substrate. The flexible circuit 700 is advantageously long, thin and narrow. Often, the flexible substrate 705 utilizes an insulating material or a dielectric. The flexible circuit 700 has been formed into a cylindrical shape 760 over at least a portion of its length. Conductive traces 710 are formed onto substrate 705. For example, a metal foil layer may be applied to or adhered to the substrate 705. Conductive traces 710 may be etched from this foil layer. The conductive traces 710 may be formed of a metal such as silver or copper, conductive inks and adhesives, conductive fiber such as carbon. They may be constructed by photolithography, conductive ink aerosol ink jet printing, sputter coating, etching, rolling, electroplating, vapor deposition or other methods known in the art.

Conductive traces 710 may be arranged on both sides of the substrate 705, or on only one side, and that side may be the inside surface 706 or the outside surface 707. At certain points there may be via holes (not shown), in the substrate 705. Conductive traces 710 on opposite sides of the substrate 705 are electrically connected through the via holes. In the via holes there is electrically conductive material on the walls. The conductive traces 710 may comprise a suitable metal, e.g. copper or an electrically conductive polymer or another electrically conductive material.

The proximal end 750 of the conductive traces 710 may be terminated in a connective means such as a solder pad 720, connectors, or similar structure as described above, for connecting the trace to other medical equipment, such as a power source, diagnostic equipment, or monitoring equipment. The proximal end 750 may also remain flat for ease of connection (as shown) The distal end 770 of the conductive traces 710 are terminated in a distal connector, such as distal solder pads 721, for example. While the majority of the flexible circuit 700 has been formed into a cylinder, the distal portion 760 may remain flat for easier connections (not shown), or may start flat and be formed into a cylinder before or after the first catheter component is attached to a second catheter component.

The second catheter component includes a flexible circuit 800, with a flexible substrate 805, typically made with a thin flexible plastic or metal foil as the substrate. The flexible circuit 800 has a length determined by the application, but is preferably shorter than flexible circuit 700 and may be even more thin and narrow. Often, the flexible substrate 805 utilizes an insulating material or a dielectric. The flexible circuit 800 has been formed into a cylindrical shape over a portion or all of its length. Conductive traces 810 are formed onto substrate 805. For example, a metal foil layer may be applied to or adhered to the substrate 805. Conductive traces 810 may be etched from this foil layer. The conductive traces 810 may be formed of a metal such as silver or copper, conductive inks and adhesives, conductive fiber such as carbon. They may be constructed by photolithography, conductive ink aerosol ink jet printing, sputter coating, etching, rolling, electroplating, vapor deposition or other methods known in the art.

Conductive traces 810 may be arranged on both sides of the substrate 805, or on only one side, and that side may be the inside or outside. At certain points there may be holes, called via holes (not shown) in the substrate 805. Conductive traces 810 on opposite sides of the substrate 805 are electrically connected through the via holes. In the via holes there is electrically conductive material on the walls. The conductive traces 810 may comprise a suitable metal, e.g. copper or an electrically conductive polymer or another electrically conductive material.

The proximal end 850 of the conductive traces 810 may be terminated in a connective means such as a solder pad 820, connectors, or similar structure as described above, for connecting the trace to traces on the first catheter component or other equipment. The distal end of the conductive traces 810 are either terminated in a sensor, electrode 880, or in a distal connector, such as distal solder pads (see, e.g., 121), for example. While the majority of the flexible circuit 800 has been formed into a cylinder, the portions may remain flat for easier connections, or may start flat and be formed into a cylinder before or after another catheter component is attached to a second catheter component.

The second catheter component may be formed into various shapes. For example, as shown in FIG. 15A, it may be shaped into a lasso style, with sensing electrodes 880 arranged at intervals around the lasso and on the shaft. The catheter shown in FIG. 15A may comprise, for example, three catheter components (a longer shaft, a bend region, and a lasso region), two catheter components (for example, a longer shaft and a lasso region, with the bend present on one or the other, or forming the joint) as shown in FIG. 15A, or one catheter component that forms all three regions, for example without solder or connections.

For ease of display, FIG. 15A shows a lasso style catheter with two electrodes. However, due to the ease of construction the present invention supplies, the ability to provide a highly dense trace pattern (numerous traces in a small package) without increasing manufacturing cost, and the robust durability of the present invention, the number of electrodes is no longer limited by manufacturing or shaft space considerations. As a result, any number of electrodes may be provided, and in any pattern. For example, 10 electrodes may be provided in evenly spaced locations about the ring, or about the ring and the shaft. Likewise, 20 electrodes may be provided in 10 pairs of two. While ring electrodes are pictured, the electrodes could advantageously be numerous spot electrodes, with some electrodes on the outside of the component, such that they would contact the tissue being treated or studied, and other electrodes on the inside of the ring inside the blood pool.

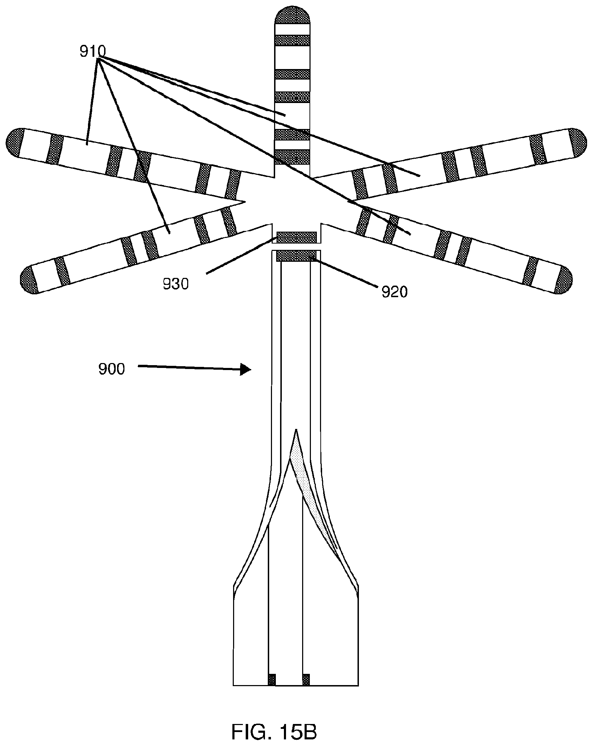

As shown in FIG. 15B, a catheter may have, in effect, numerous "second catheter components" 910 that each branch off the first catheter component 900. The second catheter components may be joined at the proximal ends into a common connector 930 that connects to the first catheter at its connector 920. Alternatively, the second catheter components may each have a connector 930 and individually connect to the connector(s) 920 (not shown). Each of these catheter components is formed as in any of the embodiments above. In one embodiment, shown in FIG. 15B above, the numerous second catheter components 910 include a flexible circuit with a flexible substrate typically made with a thin flexible plastic or metal foil as the substrate. The flexible circuit is advantageously long, thin and narrow. Often, the flexible substrate utilizes an insulating material or a dielectric. The flexible circuit has been formed into a cylindrical shape. Conductive traces are formed onto substrate. For example, a metal foil layer may be applied to or adhered to the substrate. Conductive traces may be etched from this foil layer. The conductive traces may be formed of a metal such as silver or copper, conductive inks and adhesives, conductive fiber such as carbon. They may be constructed by photolithography, conductive ink aerosol ink jet printing, sputter coating, etching, rolling, electroplating, vapor deposition or other methods known in the art.

As with FIG. 15A due to the ease of construction the present invention supplies, the ability to provide a highly dense trace pattern without increasing manufacturing cost, and the robust durability of the present invention, the number of electrodes is not limited by manufacturing or shaft space considerations. As a result, any number of electrodes may be provided on each second catheter element, and in any pattern. For example, as shown each catheter component has 6 electrodes, or 3 pairs. This is for example only, and in practice the component may have 1 electrode, e.g., at its tip, or any other number of electrodes. While ring electrodes are pictured, the electrodes could advantageously be numerous spot electrodes, with some electrodes on each side of the component, such that there would always be some electrodes in contact with the tissue being treated or studied, and potentially other electrodes inside the blood pool.

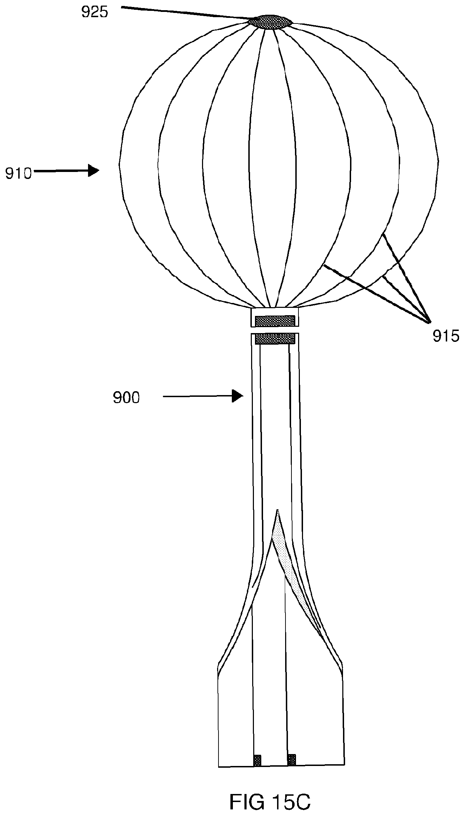

As shown in FIG. 15C, a catheter may also have, in effect, a second catheter component(s) 910 that is in the shape of a basket catheter. In this design, the second catheter component includes numerous arms or splines 915. The second catheter component arms 915 may be joined at the proximal ends into a common connector 930 that connects to the first catheter at its connector 920. Alternatively, the second catheter components may each have a connector 930 and individually connect to the connector(s) 920 (not shown). The arms 915 are joined at the distal end in cap 925. Each of these catheter components is formed as in any of the embodiments above. So, in one embodiment, shown in FIG. 15C, the second catheter component 910 include a flexible circuit with a flexible substrate typically made with a thin flexible plastic or metal foil as the substrate. The flexible circuit is advantageously long, thin and narrow. Often, the flexible substrate utilizes an insulating material or a dielectric. The flexible circuit has been formed into a cylindrical shape. Conductive traces are formed onto substrate. For example, a metal foil layer may be applied to or adhered to the substrate. Conductive traces may be etched from this foil layer. The conductive traces may be formed of a metal such as silver or copper, conductive inks and adhesives, conductive fiber such as carbon. They may be constructed by photolithography, conductive ink aerosol inkjet printing, sputter coating, etching, rolling, electroplating, vapor deposition or other methods known in the art.

As with FIG. 15A due to the ease of construction the present invention supplies, the ability to provide a highly dense trace pattern without increasing manufacturing cost, and the robust durability of the present invention, the number of electrodes is not limited by manufacturing or shaft space considerations. As a result, any number of electrodes may be provided on each second catheter element, and in any pattern. For example, common mapping catheters such as the Boston Scientific Constellation ^{™} or St. Jude's EnSite Array ^{™} may traditionally include 64 electrodes and are manufactured in a highly laborious process. However, it has been recognized that additional electrodes would be advantageous to both the speed and accuracy of a mapping procedure. As a result, it is highly desirable to provide a mapping catheter with 128 or more electrodes. However, such a catheter is difficult to manufacture and provide quality control under present manufacturing techniques. The present invention solves this dilemma by providing a method of reliably and efficiently manufacturing a high density mapping catheter.

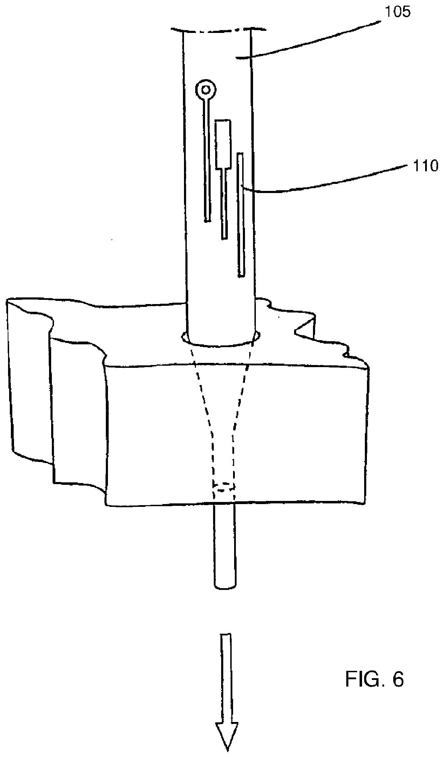

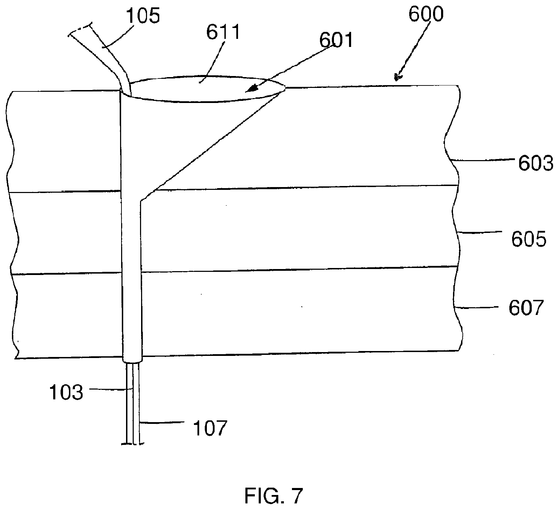

FIGs. 6 and 7 show a method for forming the flexible circuit 100 into a substantially cylindrical shape. Generally, when manufacturing the flexible circuit 100, an elongated substrate 105 is at least partly brought into a cylindrical shape along all or a portion of its length and the inside of the cylindrically shaped flexible circuit 100 may be at least partly sealed from the outside. The flexible circuit 100 has at least one electrical conductor 110 on one or both sides of the substrate 105 and may advantageously be equipped with at least one medical device element. It may be an advantage to mount medical device elements on the inside of the flexible circuit 100 but it may also be advantageous to mount the medical device elements to the outside, especially if it is integrated in the flexible substrate 105. Advantageously, the medical device elements can be mounted on the substrate 105 before the flexible circuit 100 is formed into a cylindrical shape.

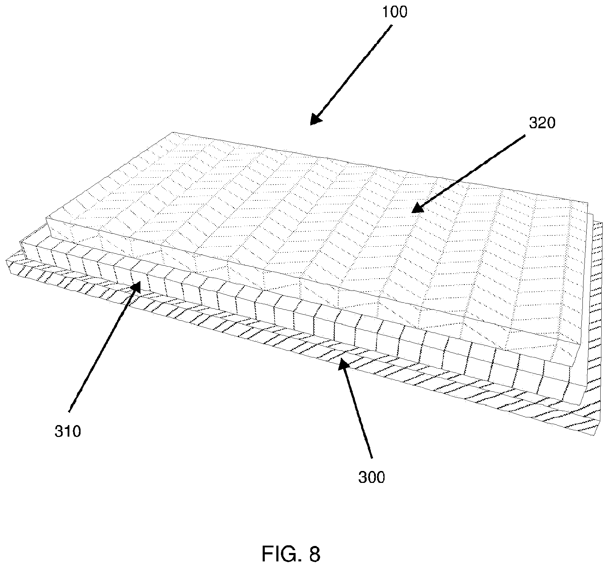

The elongated substrate may be formed by numerous methods. For example, as shown in FIG. 8, and as well known in the art, flexible circuit 100 may comprise a base layer 300 such as a polyimide film. The base layer 300 will be as thick as required for the device, but is preferably relatively thin, in the range of 5 um to 125 um, preferably 10-20 um. A number of different materials are suitable for base layers, such as polyester, Polyethylene terephthalate (PET), polyethylene napthalate (PEN), Polyetherimide (PEI), along with various fluoropolymers such as (FEP) and copolymers. While polyester films are the most cost effective, polyimide films are often preferred in a medical device setting due to their blend of electrical, mechanical, chemical and thermal properties. Flexible circuit 100 may then have an adhesive layer 310, which is typically selected for its ability to bond the base layer to the electrical layer, and for its thermal properties (e.g., ability to retain a bond in the operational temperature range). Many flex circuits use adhesive systems from the different polymer families, including polyimide adhesives, polyester adhesives, acrylics, epoxies, and phenolics. The adhesive may be a thermoset or a thermoplastic adhesive. As with the base films, adhesives come in different thickness. Thickness selection is typically a function of the application.

The flexible circuit 100 then has a conductive layer 320, such as copper foil layer 320. In traditional flexible circuit manufacturing methods, the adhesive layer 310 is needed as conductive layer 320 will not adhere well enough to the polyimide film 300 for a medical device. The conductive traces 110 (not shown in FIG. 8) are etched into this conductive layer 320. There are a wide variety of metal foils of varying thickness that are suitable, such as gold, aluminum, nickel, or silver. Likewise, a conductive polymer thick film process may be used to screen print or stencil conductive inks on to the base layer.

Copper foils are preferred due to their balance of cost, physical, and electrical performance attributes. There are many different types of copper foil, e.g., wrought, annealed, electrodeposited, or electroplated, and the type chosen is dependent on the needs of the device. In manufacturing flexible circuit 100 it is preferred that a thin surface treatment is applied to one side of the foil to improve its adhesion to the base film. Often, a protective cover lay or cover coat (not shown) is applied to protect the surface.

Once the flexible circuit 100 is formed with the base layer 300, the adhesive layer 310, and the conductive layer 320, it typically is cleaned prior to further processing or treated to remove any anti-tarnish treatment, e.g. via acid washing or acid etching. Next a pattern is prepared using, e.g., screen printing or photo imaging. Once the desired resist pattern is applied to the laminate by the chosen process, the exposed conductor is removed via etching. The resist pattern material is chosen to be impervious to the selected etching material. After etching, the resist material is removed, leaving the circuit.

It is of course understood that while flexible circuit 100 is depicted with one each base layer, adhesive layer, and foil layer, it may in practice have any number or combination thereof. While multiple layers do increase manufacturing costs, they also increase circuit density which may be necessary for a diagnostic balloon catheter, for example, which may desirably include hundreds of electrodes. In addition, via holes may be formed in one or in multiple layers, as described above, for creating connections between different trace layers, sensors, or any combination thereof.

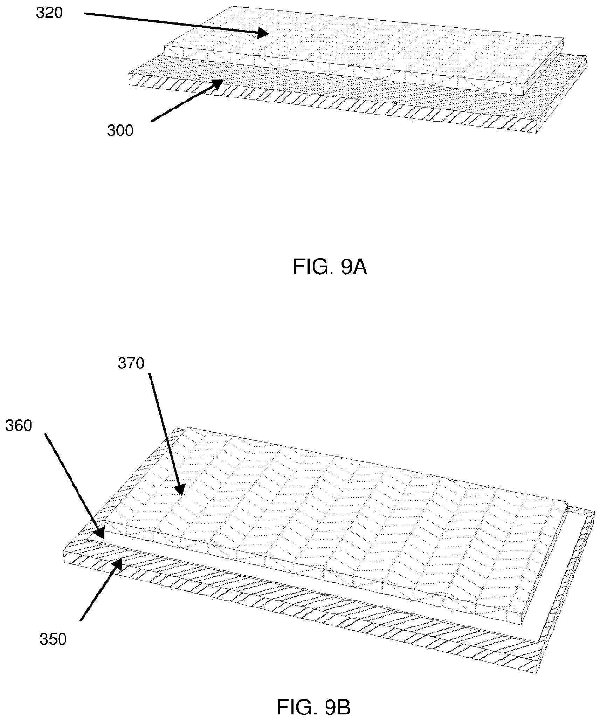

In the context of the present invention it is advantageous to make the flexible circuit 100 as thin as possible. Because an adhesive layer may add 20 um to the thickness, it is desirable to eliminate this layer, if sufficient adhesion between the base layer 300 and the conductive layer 320 can be achieved by other means. Thus, as shown in FIG. 9A, the flexible circuit 100 comprises base layer 300 and conductive layer 320, and is substantially thinner than the prior art flexible circuits.

Thus, the flexible circuit of FIG. 9A may be manufactured by heat laminating the conductive layer 320 to the base layer 300 using heat and pressure to bond, for example, a copper foil to, for example, a highly bondable polyimide layer. In such an embodiment it is preferred that the base layer 300 be a polymer with exposed functional groups that will adsorb or adhere to the conductive layer. For example preferred groups include alkanes, unsaturated monomers, aromatic ketones, aromatic aliphatic ketones, carboxylic groups or carboxylic anhydride groups, amino groups, or one or more nitrogen functional groups, or another group that will adsorb or chelate a metallic ion or colloidal structure. Preferably an ultra-thin layer of metal is first formed on the base layer surface, a seed layer, often through sputtering, and the layer may be from a couple angstroms thick to 1 um. In some cases this seed layer may have sufficient electrical properties to act alone as the flex circuit conductor. However, generally after the sputtering layer is added, a more substantial layer of metal is added through, e.g., metal vapor deposition, to build the conductive layer to the required thickness. The flexible circuit 100 may be made through a combination of additive photolithographic, plating, electroforming and other technologies. Examples of different types of metals used in a film metallization process are copper, aluminum, gold, silver, nickel, chromium, magnesium, zinc and alloys containing two or more metals.

The conductive layer 320 consists of one or more very thin layers formed by a sputtered metal on which a thicker metal is generally plated or otherwise added. The circuit lines are created in an additive process by preferably sputtering a positively charged metal to a negatively charged base. If needed a photoresist mask is applied, either before the sputtered layer or on top of the sputtered layer, and the conductive layer is applied on top of the seed (or sputtered) layer only where the non-conductive photoresist has left it exposed. Additional metal is then plated onto the exposed seed lines. Excess seed material is etched away. In the case of a multi-layer flexible circuit, the process may be repeated.

There are many methods of adhesivelessly applying metal to a base layer. While others are within the scope of this invention a couple will be discussed here. First, copper may be applied via vapor deposition. Copper is vaporized in a vacuum chamber and the metal vapor is deposited onto the base layer. In some cases, a surface treatment on the film enhances the copper adhesion. While a very thin layer of copper is deposited via this method, additional copper can be added by electrolytic plating. Second, copper may be applied by sputtered deposition. The base layer is again placed in a vacuum chamber a copper cathode. The cathode is bombarded with positive ions causing small particles of the copper to impinge on the film. Additional thickness may be added by electrolytically plating copper. A base metal of chrome or nickel may enhance performance.

Copper may also be added via chemical deposition. A base layer is roll processed through electroless metal chemistries to produce a seed layer. Additional thickness may be added by electrolytically plating copper. Copper may also be added by solution casting. A liquid solution of polyamic acid is cast onto the copper foil. It is subsequently heated to a point where the solvent is evaporated off leaving a polyimide (or amide) film.

In one embodiment, shown in FIG. 9B, the flexible circuit comprises base layer 350, a seed layer 360, and a conductive layer 370. In this embodiment a thin coating of conductor material is sputter coated or electrolessly plated onto the base layer. In a preferred embodiment a roughly 1 um copper layer 360 is sputtered onto a thermoplastic polymer base layer 350. Once the seed coating is applied to form the seed layer 360, an additional layer is electrodeposited up to the required thickness for the circuit to provide conductive layer 370. If necessary the flexible circuit is etched to remove any excess or errant copper connecting the desired circuit traces. This process has several advantages, including less waste, environmental suitability (due to reduced etching), extremely thin conductive traces and most importantly a smaller, thinner flexible circuit on the order of 0.5 to 50 um, preferably 10 to 20 um, with thicknesses as small as 0.1 um possible.

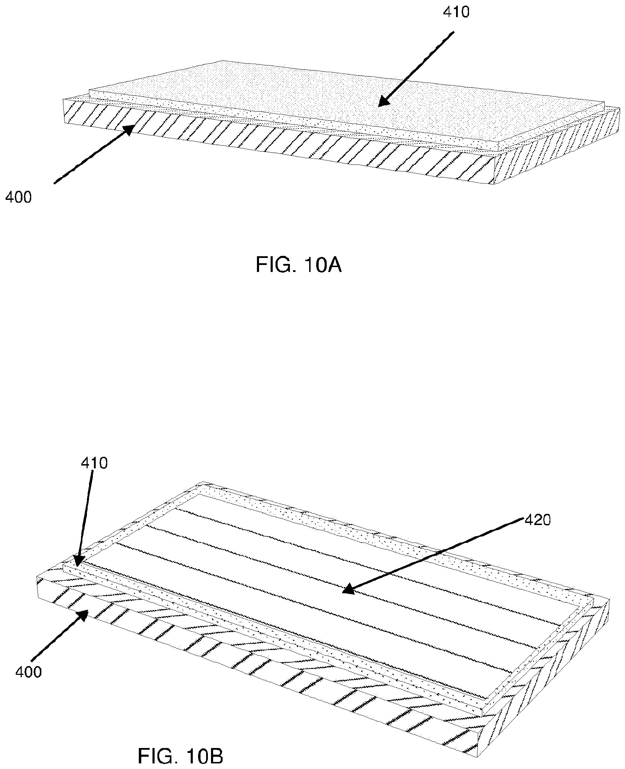

In another preferred embodiment, shown in FIGs. 10A-C, the flexible circuit is formed of a base layer 400, a monomer layer 410, a seed layer 420, and a conductive layer 430. This embodiment has the advantage of additively coupling the seed layer 420 to the base layer in a much more robust and strong fashion, as explained below. It further has the advantage of allowing wider choice in substrates, such that it is particularly advantageous for use with a thermoplastic base layer. Traditional flexible circuit technology typically uses a thermoset polyimide as the base layer. These materials are difficult to handle in a medical device setting, which often relies on thermoplastic materials that can be reflowed into a desired shape or form. This embodiment is particularly effective in utilizing a thermoplastic material such as a thermoplastic Acrylic, Acrylonitrile butadiene styrene (ABS), Nylon, Polylactic acid (PLA), Polybenzimidazole (PBI), Polyurethane, Polycarbonate (PC), Polyamide, Polyethylene (PE), Polypropylene (PP), Polystyrene (PS), Polyvinyl chloride (PVC), thermoplastic Polyimides, Polyolefins, Polyether ether ketone (PEEK), Fluoropolymers, Polytetrafluoroethylene (PTFE), Polyethers, such as a polyether block amide, e.g., Pebax^{®} as the base layer. It is also preferred that the material be capable of being made radiopaque for use with x-ray and fluoroscopes.

As a result, the flexible circuit is both formed of a thermoplastic that may be reflowed, shaped, or formed as many traditional catheters and guidewires are, and yet retains a strong bond to the electrical traces as found in traditional adhesively bound thermoset polyimide flexible circuits. This is key for many medical devices, which undergo a wide range of temperatures during use, especially during ablation or the like, but are also subject to a wide range of physical stresses such as bending, twisting, kinking, and the like, all of which may stress or destroy the bond between the electrical trace and the base layer. Providing a proper bond that allows the even application of stress to the electrical trace is vital for a reliable durable medical device. Providing a thermoplastic substrate that may be reflowed to meld fully to a lumen body in some embodiments is another advantage of this embodiment. Providing a thermoplastic substrate that may be reflowed into a sealed cylindrical shape without the use of glues, joints, or welding is yet another advantage.

With reference to FIG.10A, in this embodiment construction of the flexible circuit begins with a base layer 400. The base layer 400 is preferably a base layer material that includes extractable hydrogen atoms such that the base layer 400 is suitable for copolymerization. In the event that a preferred base material does not include sufficient extractable hydrogen atoms or another polymerization mechanism, it may be coated with a suitable coating that provides the hydrogen atoms, such as parylene. A polymerizable monomer layer 410 is added to the base layer, and a portion of the monomers are covalently bonded to the base layer 400, preferably via hydrogen extraction.

There are many ways to accomplish this step. For example, the monomer layer 410 may be evenly applied over an entire portion of the base layer. An initiator (not shown) may be applied, either with the monomer layer or separately. The monomer layer 410 is typically applied as a monomer in a solvent(s). The solvent selected will depend on the monomer, compatibility with the base layer (e.g., will not damage the base layer), and the initiator used, if any. Suitable solvents include water, methanol, ethanol, acetone, ethyl acetate, or ethylene glycol.

Either with or without an initiator, the monomer layer 410 is covalently bonded to the entire portion of base layer 400. While an adhesive layer is ordinarily measured in micrometers, the monomer layer 410 can have a thickness between 5 and 500 pm, or preferably from 10 to 100 pm, and accordingly allows for a much thinner medical device. It is noted that only a portion of the monomers in the monomer layer will be polymerized in a typical application, as typically there will be some individual monomers that will not react. Unreacted monomers are preferably removed prior to further processing to avoid reducing the yield in the steps below, e.g., via a suitable water or solvent bath. Preferred monomers include alkanes, unsaturated monomers, aromatic ketones, aromatic alifactic ketones, or more preferably monomers with one or more carboxylic groups or carboxylic anhydride groups, e.g., methacrylic acid, acrylic acid, maleic acid, nadic anhydride, a tetracarboxylic acid such as pyromellitic dianhydride (PMDA) or 3,3',4,4'-biphenyltetracarboxylic dianhydride (BPDA), tert-butyl acrylate, glycidyle acrylate, glycidyl methacrylate, nadic anhydride, phenylethynyl phthalic anhydride (PEPA), acrylonitrile, vinyl acetate, styrene, maleic anhydride, iminodiacetic acid, methacrylic anhydride and acrylic anhydride, monomers with one or more amino groups, or one or more nitrogen functional groups, or another monomer that will adsorb or chelate a metallic ion or colloidal structure. Different monomers will require different reaction conditions. For example, certain monomers may prefer or require a specific initiator and a specific pH range.

In a different embodiment, FIG. 10B, the monomer layer 410 may be evenly applied over an entire portion of the base layer 400, and an initiator may also be applied, as above. A desired conductor pattern is developed, and a mask or resist pattern 420 is applied over the base layer/monomer 400/410. The initiator is actuated, e.g., by excitation of the initiator in the non-masked portions, and the monomers are bonded to the base layer in either the desired conductor pattern or in a negative thereof. In a preferred embodiment the initiator is a photo initiator, when activated by irradiation with light provides a free radical, so that the free radical initiates a polymerization reaction between the monomers and preferably the base layer, preferably removing a covalently bound hydrogen atom to make room for the bond. While many different initiators can be used, the initiator is preferably a radical initiator, such as organic and inorganic peroxides, halogens, or azo compounds. More preferably the initiator is a photo initiator such as antraquinone, thioxanthone, isopropyl thioxanthone, chlorothioxanthone, xanthone, benzophenone, camphorquinone, benzophenone, naphthalene, anthraquinone, acetopheonone, benzoyl dimethylketal, formaldehyde, aldehydes, acetone, acetophenone and fluorenone and their derivatives. The initiator is actuated via irradiation with light. While many light sources are suitable, a precise focused light source, e.g. a laser, is preferred. The frequency of the laser and its power is matched to the initiator in use. The application of energy via laser allows for a very precise covalent bonding of monomers to the substrate in a precise pattern suitable as a base for very small traces, e.g., 100 pm wide. The size of the traces will be determined by the needs of the trace, e.g., how much current it must carry, needed fatigue life, device size limitations, and how physically robust it must be. Preferred trace widths include from 20 um to 200um, preferably from 5-20 um.

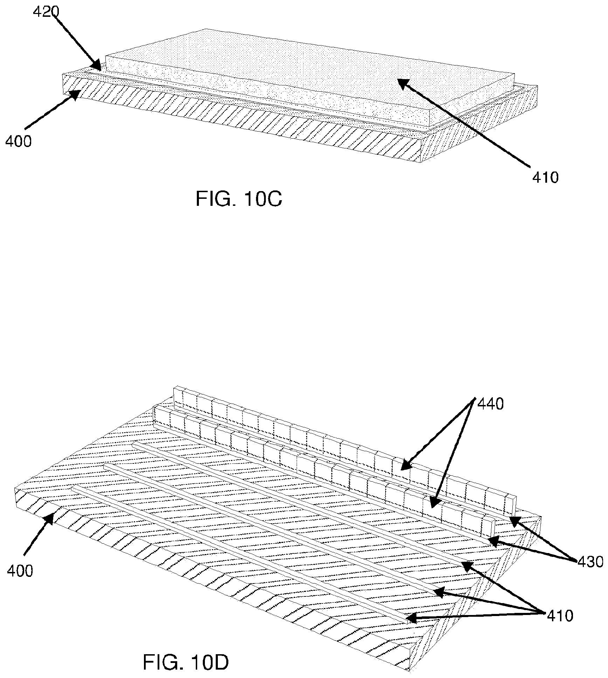

Finally, in a third embodiment, FIG. 10C, a desired conductor pattern is developed, and a mask or resist pattern 420 is applied over the base layer 400. The monomer layer 410 may be applied over (1) both the unmasked portion and the mask or resist pattern (as shown in FIG. 10C), or (2) over only the unmasked portion. The monomer layer 410 is then initiated, and the monomers are covalently bonded to the base layer in the desired conductor pattern. The monomers over the masked portion either remain unbonded, or are bonded to the mask and removed with it.

Once the monomer layer 410 is covalently bonded to the base layer 400 a seed layer 430 (FIG. 10D) is applied. The seed layer 430 comprises seed material such as a metal, metal nanoparticles, colloidal metal, or preferably metal ions. Alternatively, the monomer layer may further include monomers with conducting groups, such that the monomer layer and the seed layer are one and the same.

The precise manner of applying the seed layer will depend on the manner in which the monomer was bonded to the base layer, above. For example, in a base layer 400 prepared as in FIG. 10A, the seed layer 430 may be applied over a mask so that the seed material is only applied in the desired conductive trace pattern. In the embodiments of FIG. 10B or 10C the seed material may be applied over a mask as in 10A, or it may be applied over the entire surface, as it will primarily bond to the pattern formed by the monomer layer 410. Once applied, if the seed material is an ionic metal, it must then be reduced.

Preferred seed materials must adsorb to or adhere strongly to the monomer layer 410, and are further selected for their ability to adhere to the conductive trace layer 440. Examples include metallic ions, metallic colloidal structures, or a dispersion of colloidal metal particles. Suitable examples include palladium, ruthenium, rhodium, osmium, iridium, platinum, silver, copper, and their ions. In some embodiments the seed materials can be applied to the base layer 400 at the same time as the monomer layer and/or the initiator. In other embodiments the pH needed for optimal ion adsorption is elevated (e.g., above 7, or above 10), while the pH needed for optimal polymerization of the monomer to the base layer 400 is low (e.g., below 4), and accordingly the polymerization must occur, the substrate must be washed, the pH raised, and then the metal ions applied.

In other embodiments the seed materials are applied by dipping the base layer 400 and monomer layer 410 in a bath of the metallic ions. Once the metallic ions are suitably adhered to the monomer layer 410 and the base layer 400, they are reduced by dipping in a reducing media and washed.

Once the seed layer 430 is applied, the conductive trace layer 440 is applied to the seed layer. For example, the base layer 400/monomer layer 410/seed layer 430 is dipped into a copper bath. The length of dipping time depends on the desired thickness of the conductive traces 110. While copper is one preferred conductive trace 110 material, other suitable metals include silver, gold, nickel, titanium and chromium. Additional metal layers are applied as needed.

Subsequently, the flexible circuit 100 is subjected to post processing, such as etching to remove excessive or misplaced conductive portions via processes well known in the art. The flexible circuit 100 may have a third metal or a masking layer applied on top of the conductive trace material in the same or a different pattern. The seed material and the conductive trace material outside this third metal or masking layer may be removed.

In a particularly advantageous embodiment of the present invention, base layer 400 is already formed into a cylinder (as described above) or another three dimensional shape prior to the completion of the steps needed to form a completed flexible circuit. For example, base layer 400 may be formed into a cylinder, reflowed to join the edge joints 165 to each other, and then may have the monomer layer 410, the seed layer 430, and the conductive layer 440 subsequently applied and as necessary cleaned up with etching. Likewise, one or more steps can be performed prior to the formation of the cylinder. The monomer layer 410 and the initiator may be added to the base layer 400. The initiator may be initiated and the monomer layer covalently bonded to the base layer. At this point the cylinder may be formed, and then the seed layer 430 and the conductive layer 440 may be applied. Processing in this manner has the advantage of applying the conductive trace to the already formed cylinder, greatly reducing the stress the conductive traces would have experienced during processing.

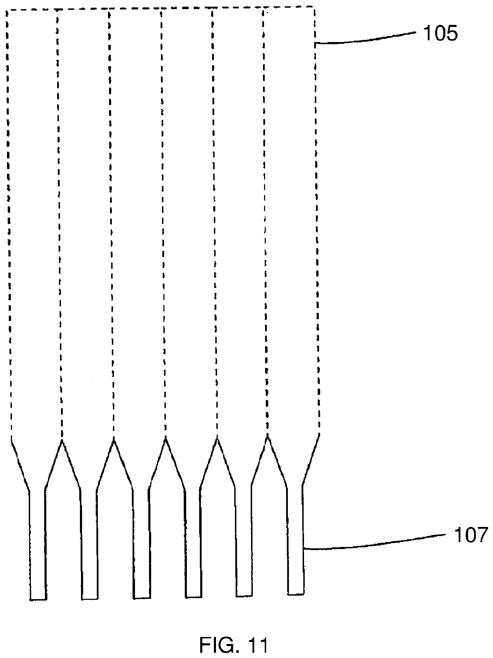

In one embodiment the flexible circuit 100 is provided with a tip 107 (see FIG. 11) on at least one of the ends of the flexible circuit 100. The tip 107 is narrower than the rest of the support member and may have a length of approximately 10 to 50 mm, preferably 15 to 30 mm. When forming the flexible circuit 100 at least partly into a cylindrical shape, a jig or tool 600 made out of a block of material like metal or plastic is used. The metal used may for example be steel, brass, copper or any other alloy. A suitable plastic may for example be polymethacrylate, known as Plexiglass^{™}.

The jig or tool 600 is provided with a small hole 601 having a funnel-like opening 611. The hole 601 and the funnel-like opening 611 are adapted not to damage the flexible circuit 100 or the other elements provided on the flexible circuit 100. For example may a lining be provided in the hole 601 and/or funnel-like opening 611.

The tip 103 of the flexible circuit 100 is threaded through the funnel-like opening 611 and the small hole 601. The opening 611 is filled with an adhesive or glue. If substrate 105 is a polyimide, it may be advantageous to use PolyCaproLacton (PCL) which has a good adhesion to polyimide. The adhesive or glue may be distributed by means of a dispenser. Generally, an adhesive is selected that has a good adhesion to the material of the flexible substrate 105. The adhesion between the adhesive and the flexible substrate 105 needs to be good to maintain the flexible substrate 105 in a tube shape. The adhesive is melted and fills the flexible substrate 105. When the flexible substrate 105 is pulled through the lower part of the hole, it is cooled and the PCL crystallizes (it becomes solid) and forms a reinforcing or rigidifying element 103. The reinforcing or rigidifying element 103 may comprise the solidified adhesive material, a separate reinforcing or rigidifying element or a combination of the solidified adhesive material and the separate reinforcing or rigidifying element.

If there are via holes 140 in the flexible substrate 105 these will filled with adhesive material as the flexible substrate 105 being fed through the tool 600. The adhesive material will fill the via holes 140 completely and will substantially be in line with the outside surface of the flexible substrate 105. If the flexible circuit 100 is not covered with a biocompatible material, like a biocompatible hydrogel, it is advantageous that the adhesive material used is biocompatible. Further details can be found in United States Patent Publication No. US20090143651, published June 4, 2009.

It is as well possible to use welding, for example laser welding, to weld the adjacent edges of the flexible circuit 100 to each other. In this case the jig or tool 600 may be provided with welding equipment that welds the edges of the flexible circuit 100 to each other as the substrate 105 is drawn through the jig or tool 600. In this case the flexible circuit 100 may also be provided with a separate reinforcing or rigidifying element 103 as the substrate 105 is drawn through the jig or tool 600. The reinforcing or rigidifying element 103 may advantageously be provided on the inside of the cylindrical flexible circuit 100.

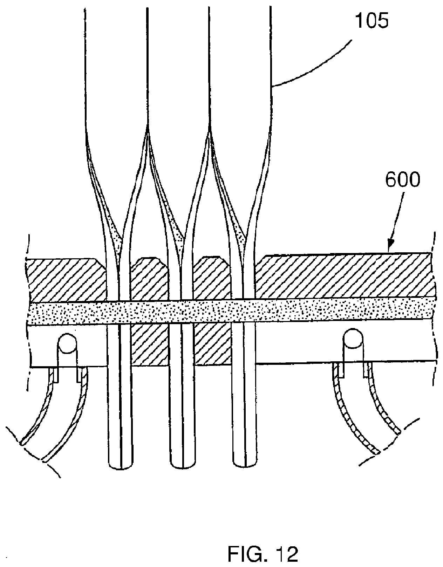

It is possible to produce medical devices in great numbers efficiently. The flexible circuit 100 may be manufactured simultaneously in great numbers. In one example, shown in FIGs. 11-13, flexible circuits 100 are manufactured from sheets or panels of a suitable material. Common widths of the panels or sheets are 30 or 45 cm, which allows hundreds of flexible circuits 100 (approximately 1-2 mm wide) to be manufactured simultaneously. The flexible circuits 100 are separated by perforations (done for example by milling or laser ablation) to make it easy to separate them. This allows simultaneous formation of several flexible circuits 100 as depicted in FIG. 12.

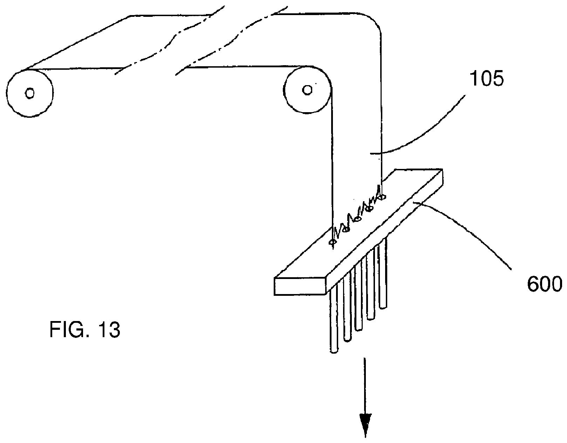

It is also possible to make the production continuous as indicated in FIG. 13. The flexible circuits 100 are preferably separated from one another by a suitable perforation or other suitable technologies. The perforation may be added before the perforated sheet or panel enters the tool or jig 600. Here two standard methods are combined with the flexible circuit 100 described herein in a continuous production process. First, the substrate 105 is subjected to standard process steps used today by manufacturers of flexible printed wire boards, such as via drilling, pattern formation by lithography and etching. Conductors may also be formed by ink jet printing or in other ways. Next, the medical device elements are attached by standard pick-and-place equipment using conducing glue, soldering or some other method. Finally, the sheet or panel is fed into a tool or jig 600 with several parallel holes 609 with funnel-shaped openings 611. The feeding mechanism is omitted in the figure. This would constitute a fully continuous process. Flexible circuits 100 can be cut off in batches after passage of the tool or jig 600.

One advantage with the device described herein is that the construction is relatively simple. Thereby reliability can be improved. Basically the flexible circuit 100 itself constitutes a device suitable for invasive use. Since the construction is relatively simple the device may also be manufactured relatively inexpensively which facilitates the use of the device as a single use article.

The manufacturing process brings advantages for example in terms of automation. The manufacturing process is also easy to implement in a bigger scale since several devices can be manufactured in parallel.

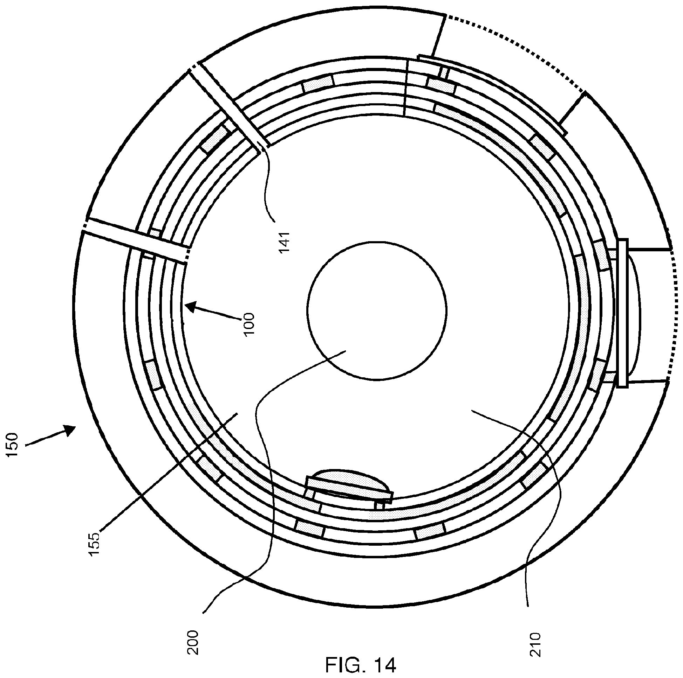

FIG. 14 shows a cross section according to an example. In manufacturing flexible circuit 100 is formed with a closed lumen. Flexible circuit 100 is formed on a removable carrier 200 with its lumen filled with adhesive 210 to seal it into a cylindrical form. Manufacturing the structure in FIG. 3 may be accomplished by first mounting the flexible circuit 100 into or onto a carrier 200, then inserting the carrier 200 into the open lumen 155 then mounting the flexible circuit 100 to the open lumen 155. Carrier 200 and adhesive 210 are then removed, leaving lumen 155 open. The carrier 200 removal may be facilitate by heating the adhesive, by having a lubricious PTFE surface on the carrier, or pulling on the carrier such that it elongates in and reduces in diameter, for example. Irrigation ports 141 maybe mechanically formed, or created by use of a laser, before or after the flexible circuit 100 is mounted.

The medical device of the present invention is defined by appended independent claim 1. Dependent claims disclose exemplary embodiments.

## Claims

1. A medical device (151, 152), comprising:
an elongated lumen shaft comprising:
a shaft body having a thermoplastic polymeric cylindrical shaft wall (150, 154, 157);
a cylindrical flexible circuit (100) comprising:
a dielectric layer (105);
a conductive trace layer (110); the conductive trace layer (110) comprising individual electrical conductor lines (110) coupled to a medical device element (125, 130, 160) at a distal end of the electrical conductor lines; and
an open, unfilled lumen (155),
**characterized in that** it comprises:
the thermoplastic polymer dielectric layer (105) in a cylindrical form;
the thermoplastic polymer dielectric layer (105) comprising a bendable and
stretchable substrate at least partly attached to the thermoplastic polymeric cylindrical shaft wall (150, 154, 157) via a melted connection between the thermoplastic polymeric cylindrical shaft wall (150, 154, 157) and the thermoplastic polymer dielectric layer (105), the melted connection comprising a portion of the wall where a first polymer from the thermoplastic polymeric cylindrical shaft wall and a second polymer from the thermoplastic polymer dielectric layer are mixed together;

2. The medical device of claim 1, wherein the conductor lines (110) are shaped in such a way as to be stretchable.

3. The medical device of claim 1, wherein the bendable and stretchable substrate (105) is capable of assuming various bends and motions.

4. The medical device of claim 1, wherein the cylindrical flexible circuit (100) further comprises a second flexible circuit (105) that is joined to a first flexible circuit (105).

5. The medical device of claim 1, further comprising vias (140) in the dielectric layer electrically connecting features on both sides of the dielectric layer.

6. The medical device of claim 4, wherein the second flexible circuit (105) is shorter than the first flexible circuit (105).

7. The medical device of claim 1, wherein the elongated lumen shaft (150) has an outer diameter that varies over its length.

8. The medical device of claim 1, wherein the flexible circuit (100) has a thickness less than 50 microns.

9. The medical device of claim 8, wherein the medical device element (125, 130, 160) is embedded in the flexible circuit (100).

10. The medical device of claim 1, wherein the cylindrical flexible circuit (100) further comprises a reflowed joint (165) joined by reflowing a thermoplastic dielectric between first and a second flexible circuit edge.

11. The medical device of claim 1, wherein the medical device element is an electrode (130) located on an exterior of the cylindrical flexible circuit (100), and further comprising an electrical connection between the electrode and the conductive trace layer (110) through a via (140).

12. The medical device of claim 1, wherein the medical device element (125, 130, 160) is located on an interior of the cylindrical flexible circuit, and further comprising a second medical device element (125, 130, 160) located on an exterior of the cylindrical flexible circuit (100).

## Patentansprüche

1. Medizinische Vorrichtung (151, 152), umfassend:
einen länglichen Lumenschaft, umfassend:
einen Schaftkörper mit einer thermoplastischen polymeren zylindrischen Schaftwand (150, 154, 157);
eine zylindrische flexible Schaltung (100), umfassend:
eine dielektrische Schicht (105);
eine leitfähige Leiterbahnschicht (110); wobei die leitfähige Leiterbahnschicht (110) einzelne elektrische Leiterbahnen (110) umfasst, die mit einem medizinischen Vorrichtungselement (125, 130, 160) an einem distalen Ende der elektrischen Leiterbahnen gekoppelt sind; und
ein offenes, ungefülltes Lumen (155),
**dadurch gekennzeichnet, dass** dieses Folgendes umfasst:
die thermoplastische polymere dielektrische Schicht (105) in einer zylindrischen Form;
die thermoplastische polymere dielektrische Schicht (105), umfassend ein biegbares und dehnbares Substrat, das mindestens teilweise an der thermoplastischen polymeren zylindrischen Schaftwand (150, 154, 157) über eine Schmelzverbindung zwischen der thermoplastischen polymeren zylindrischen Schaftwand (150, 154, 157) und der thermoplastischen polymeren dielektrischen Schicht (105) angebracht ist, wobei die Schmelzverbindung einen Abschnitt der Wand umfasst, wo ein erstes Polymer von der thermoplastischen polymeren zylindrischen Schaftwand und ein zweites Polymer von der thermoplastischen polymeren dielektrischen Schicht miteinander vermischt sind.

2. Medizinische Vorrichtung nach Anspruch 1, wobei die Leiterbahnen (110) dehnbar geformt sind.

3. Medizinische Vorrichtung nach Anspruch 1, wobei das biegbare und dehnbare Substrat (105) in der Lage ist, verschiedene Krümmungen und Bewegungen anzunehmen.

4. Medizinische Vorrichtung nach Anspruch 1, wobei die zylindrische flexible Schaltung (100) ferner eine zweite flexiblen Schaltung (105) umfasst, die mit einem ersten flexiblen Schaltung (105) verbunden ist.

5. Medizinische Vorrichtung nach Anspruch 1, ferner umfassend Durchkontaktierungen (140) in der dielektrischen Schicht, die Verbindungsmerkmale auf beiden Seiten der dielektrischen Schicht elektrisch verbinden.

6. Medizinische Vorrichtung nach Anspruch 4, wobei die zweite flexible Schaltung (105) kürzer als die erste flexible Schaltung (105) ist.

7. Medizinische Vorrichtung nach Anspruch 1, **dadurch gekennzeichnet, dass** der längliche Lumenschaft (150) einen über seine Länge variierenden Außendurchmesser aufweist.

8. Medizinische Vorrichtung nach Anspruch 1, wobei der flexible Schaltkreis (100) eine Dicke von weniger als 50 um aufweist.

9. Medizinische Vorrichtung nach Anspruch 8, wobei das medizinische Vorrichtungselement (125, 130, 160) in die flexible Schaltung (100) eingebettet ist.

10. Medizinische Vorrichtung nach Anspruch 1, wobei die zylindrische flexible Schaltung (100) ferner eine Schmelzverbindung (165) umfasst, die durch Aufschmelzen eines thermoplastischen Dielektrikums zwischen einer ersten und einer zweiten Kante der flexiblen Schaltung zusammengefügt ist.

11. Medizinische Vorrichtung nach Anspruch 1, wobei das medizinische Vorrichtungselement eine Elektrode (130) ist, die sich an einer Außenseite der zylindrischen flexiblen Schaltung (100) befindet, und ferner eine elektrische Verbindung zwischen der Elektrode und der leitfähigen Leiterbahnschicht (110) durch eine Durchkontaktierung (140) umfasst.

12. Medizinische Vorrichtung nach Anspruch 1, wobei sich das medizinische Vorrichtungselement (125, 130, 160) an einem Inneren der zylindrischen flexiblen Schaltung befindet, und ferner umfassend ein zweites medizinisches Vorrichtungselement (125, 130, 160), das sich an einer Außenseite der zylindrischen flexiblen Schaltung (100) befindet.

## Revendications

1. Dispositif médical (151, 152), comprenant :
une tige à lumière allongée comprenant :
un corps de tige ayant une paroi de tige cylindrique en polymère thermoplastique (150, 154, 157) ;
un circuit flexible cylindrique (100) comprenant :
une couche diélectrique (105) ;
une couche de trace conductrice (110) ; la couche de trace conductrice (110) comprenant des lignes conductrices électriques individuelles (110) couplées à un élément de dispositif médical (125, 130, 160) au niveau d'une extrémité distale des lignes conductrices électriques ; et
une lumière ouverte et non remplie (155),
**caractérisé en ce qu'**il comprend :
la couche diélectrique en polymère thermoplastique (105) sous une forme cylindrique ;
la couche diélectrique en polymère thermoplastique (105) comprenant un substrat pliable et étirable fixé au moins en partie à la paroi de tige cylindrique en polymère thermoplastique (150, 154, 157) par l'intermédiaire d'une liaison fondue entre la paroi de tige cylindrique en polymère thermoplastique (150, 154, 157) et la couche diélectrique en polymère thermoplastique (105), la liaison fondue comprenant une partie de la paroi où un premier polymère de la paroi de tige cylindrique en polymère thermoplastique et un second polymère de la couche diélectrique en polymère thermoplastique sont mélangés ensemble.

2. Dispositif médical selon la revendication 1, dans lequel les lignes conductrices (110) sont conformées de manière à être étirables.

3. Dispositif médical selon la revendication 1, dans lequel le substrat pliable et étirable (105) est capable d'assumer diverses courbures et mouvements.

4. Dispositif médical selon la revendication 1, dans lequel le circuit flexible cylindrique (100) comprend en outre un second circuit flexible (105) qui est relié à un premier circuit flexible (105).

5. Dispositif médical selon la revendication 1, comprenant en outre des trous d'interconnexion (140) dans la couche diélectrique connectant électriquement des éléments des deux côtés de la couche diélectrique.

6. Dispositif médical selon la revendication 4, dans lequel le second circuit flexible (105) est plus court que le premier circuit flexible (105).

7. Dispositif médical selon la revendication 1, dans lequel la tige à lumière allongée (150) a un diamètre extérieur qui varie sur sa longueur.

8. Dispositif médical selon la revendication 1, dans lequel le circuit flexible (100) a une épaisseur inférieure à 50 microns.

9. Dispositif médical selon la revendication 8, dans lequel l'élément de dispositif médical (125, 130, 160) est intégré dans le circuit flexible (100).

10. Dispositif médical selon la revendication 1, dans lequel le circuit flexible cylindrique (100) comprend en outre un joint refondu (165) relié par refusion d'un diélectrique thermoplastique entre un premier et un second bord de circuit flexible.

11. Dispositif médical selon la revendication 1, dans lequel l'élément de dispositif médical est une électrode (130) située sur un extérieur du circuit flexible cylindrique (100), et comprenant en outre une connexion électrique entre l'électrode et la couche de trace conductrice (110) à travers un trou d'interconnexion (140).

12. Dispositif médical selon la revendication 1, dans lequel l'élément de dispositif médical (125, 130, 160) est situé sur un intérieur du circuit flexible cylindrique, et comprenant en outre un second élément de dispositif médical (125, 130, 160) situé sur un extérieur du circuit flexible cylindrique (100).
